# EUROPÄISCHE PATENTSCHRIFT

(11) **EP 2 486 036 B1**
(45) Veröffentlichungstag und Bekanntmachung des Hinweises auf die Patenterteilung: **09.08.2017**
(21) Anmeldenummer: 10752324.3
(22) Anmeldetag: 09.09.2010
(51) Int. Cl.: C07D 471/04, C07D 471/16, C07D 487/04, C07D 487/16, C09K 11/06

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 08.10.2009 DE 102009048791
(43) Veröffentlichungstag der Anmeldung: 15.08.2012
(73) Patentinhaber: Merck Patent GmbH, 64293 Darmstadt (DE)
(72) Erfinder: PARHAM, Amir, Hossain, 65929 Frankfurt am Main (DE); PFLUMM, Christof, 60316 Frankfurt am Main (DE)
(86) Internationale Anmeldenummer: PCT/EP2010/005555
(87) Internationale Veröffentlichungsnummer: WO 2011/042107

(56) Entgegenhaltungen:
- EP-A2- 2 182 040
- WO-A1-2006/033563
- WO-A1-2006/080640
- WO-A2-2007/031165
- JP-A- 11 339 868
- JP-A- 2003 022 893
- US-A1- 2009 136 779
- US-A1- 2009 295 275
- CHEMISTRY - A EUROPEAN JOURNAL 20090525 WILEY-VCH VERLAG DEU, Bd. 15, Nr. 22, 25. Mai 2009 (2009-05-25), Seiten 5482-5490, XP002608473, DOI: DOI:10.1002/CHEM.200900097

## Beschreibung

Die vorliegende Erfindung betrifft phosphoreszierende organische Elektrolumineszenzvorrichtungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen (M. A. Baido et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, jedoch immer noch Verbesserungsbedarf, beispielsweise im Hinblick auf Effizienz, Betriebsspannung und insbesondere Lebensdauer. Dies gilt insbesondere für OLEDs, welche im kürzerwelligen Bereich, beispielsweise grün, emittieren.

Die Eigenschaften von phosphoreszierenden OLEDs werden nicht nur von den eingesetzten Triplettemittern bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen. Auch für fluoreszierende OLEDs gibt es bei diesen Materialien noch Verbesserungsbedarf.

Gemäß dem Stand der Technik werden unter anderem Ketone (z. B. gemäß WO 04/093207 oder WO 10/006680) oder Phosphinoxide (z. B. gemäß WO 05/003253) als Matrixmaterialien für phosphoreszierende Emitter verwendet. Allerdings besteht bei Verwendung dieser Matrixmaterialien ebenso wie bei anderen Matrixmaterialien noch Verbesserungsbedarf, insbesondere in Bezug auf die Effizienz und die Lebensdauer der Vorrichtung.

Aufgabe der vorliegenden Erfindung ist die Bereitstellung von Verbindungen, welche sich für den Einsatz in einer fluoreszierenden oder phosphoreszierenden OLED, insbesondere einer phosphoreszierenden OLED, eignen, beispielsweise als Matrixmaterial oder als Lochtransport-/ Elektronenblockiermaterial bzw. Exzitonenblockiermaterial oder als Elektronentransport- bzw. Lochblockiermaterial. Insbesondere ist es die Aufgabe der vorliegenden Erfindung, Matrixmaterialien bereitzustellen, welche sich für grün und rot phosphoreszierende OLEDs eignen.

Überraschend wurde gefunden, dass bestimmte, unten näher beschriebene Verbindungen diese Aufgabe lösen und zu deutlichen Verbesserungen der organischen Elektrolumineszenzvorrichtung führen, insbesondere hinsichtlich der Lebensdauer, der Effizienz und der Betriebsspannung. Dies gilt insbesondere für rot und grün phosphoreszierende Elektrolumineszenzvorrichtungen, bei Einsatz der Verbindungen als Matrixmaterial. Organische Elektrolumineszenzvorrichtungen, welche derartige Verbindungen enthalten, sind daher der Gegenstand der vorliegenden Erfindung.

Aus der WO 07/031165 sind verbrückte Triarylaminstrukturen mit ähnlicher Grundstruktur wie die nachfolgend beschriebenen Verbindungen bekannt. Verbindungen, die mit den unten aufgeführten Substituenten substituiert sind, sind dort jedoch nicht offenbart. Weiterhin werden diese Verbindungen nur als Emitter oder als Lochtransportmaterial beschrieben, nicht jedoch als Matrixmaterial für phosphoreszierende Emitter.

Aus der US 2009/0136779 sind Verbindungen mit ähnlicher Grundstruktur als Matrix für phosphoreszierende Emitter bekannt. Verbindungen, die mit den unten aufgeführten Substituenten substituiert sind, sind dort jedoch nicht offenbart.

Gegenstand der vorliegenden Erfindung ist eine organische Elektrolumineszenzvorrichtung enthaltend eine Verbindung gemäß der folgenden Formel (17) oder Formel (18) als Matrixmaterial für phoshoreszierende Emitter, 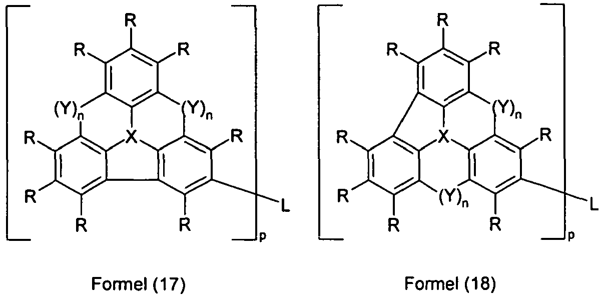 wobei für die verwendeten Symbole und indizes gilt:
- X: ist N;
- Y: ist gleich oder verschieden bei jedem Auftreten C(R)₂;
- R: ist bei jedem Auftreten gleich oder verschieden ausgewählt aus der Gruppe bestehend aus H, D, F, Cl, Br, I, CN, NO₂, N(Ar)₂, N(R¹)₂, C(=O)_{Ar}, C(=O)R¹, P(=O)(Ar)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R¹ substituiert sein kann, wobei eine oder mehrere nicht-benachbarte CH₂-Gruppen durch R¹C=CR¹, C=C, Si(R¹)₂, Ge(R¹)₂, Sn(R¹)₂, C=O, C=S, C=Se, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 80, bevorzugt 5 bis 60, aromatischen Ringatomen, das jeweils mit einem oder mehreren Resten R¹ substituiert sein kann, einer Aryloxy- oder Heteroaryloxygruppe mit 5 bis 60 aromatischen Ringatomen, die mit einem oder mehreren Resten R¹ substituiert sein kann, oder einer Kombination dieser Systeme, wobei optional zwei oder mehr benachbarte Substituenten R ein monocyclisches oder polycyclisches, aliphatisches, aromatisches oder hetero-aromatisches Ringsystem bilden können, das mit einem oder mehreren Resten R¹ substituiert sein kann;
- R¹: ist bei jedem Auftreten gleich oder verschieden ausgewählt aus der Gruppe bestehend aus H, D, F, Cl, Br, I, CN, NO₂, N(Ar)₂, N(R₂)₂, C(=O)Ar, C(=O)R², P(=O)(Ar)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R² substituiert sein kann, einer Aryloxy- oder Heteroaryl-oxygruppe mit 5 bis 60 aromatischen Ringatomen, die mit einem oder mehreren Resten R² substituiert sein kann, oder einer Kombination dieser Systeme, wobei optional zwei oder mehr benachbarte Substituenten R ein monocyclisches oder polycyclisches, aliphatisches, aromatisches oder heteroaromatisches Ringsystem bilden können, das mit einem oder mehreren Resten R² substituiert sein kann;
- R²: ist ausgewählt aus der Gruppe bestehend aus H, D, F, CN, einem aliphatischem Kohlenwasserstoffrest mit 1 bis 20 C-Atomen, einem aromatischem oder heteroaromatischem Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R² miteinander ein mono- oder polycyclisches, aliphatisches, aromatisches oder heteroaromatisches Ringsystem bilden können;
- Ar: ist bei jedem Auftreten gleich oder verschieden ein aromatisches oder heteroaromatisches Ringsystem mit 5-30 aromatischen Ringatomen, das mit einem oder mehreren nicht-aromatischen Resten R² substituiert sein kann; dabei können zwei Reste Ar, welche an dasselbe N-Atom oder P-Atom binden, auch durch eine Einfachbindung oder eine Brücke, ausgewählt aus N(R²), C(R²)₂ oder O, miteinander verbrückt sein;
- L: ist eine bivalente oder höher valente geradkettige Alkylen-, Alkyliden-, Alkylenoxy- oder Thioalkylenoxygruppe mit 1 bis 40 C-Atomen oder eine verzweigte oder cyclische Alkylen-, Alkyliden-, Alkylenoxy- oder Thioalkylenoxygruppe mit 3 bis 40 C-Atomen oder eine Alkenylen- oder Alkinylengruppe mit 2 bis 40 C-Atomen, die mit jeweils einem oder mehreren Resten R¹ substituiert sein kann, wobei eine oder mehrere nicht benachbarte CH₂-Gruppen durch -R¹C=CR¹-, -C=C-, Si(R¹)₂, Ge(R¹)₂, Sn(R¹)₂, C=O, C=S, C=Se, C=NR¹, P(=O)R¹, S=O, SO₂, -O-, -S- oder -CONR¹- ersetzt sein können und wobei ein oder mehrere H-Atome durch D, F, Cl, Br, I, CN oder NO₂ ersetzt sein können, oder ein mindestens bivalentes aromatisches oder heteroaromatisches Ringsystem mit 5 bis 80, bevorzugt 5 bis 40, aromatischen Ringatomen, welches durch einen oder mehrere Reste R¹ substituiert sein kann, oder P(R¹)₃₋ₚ, P(=O)(R¹)₃₋ₚ, C(R¹)₄₋ₚ, Si(R¹)₄₋ₚ, N(Ar)₃₋ₚ oder eine Kombination aus zwei, drei, vier oder fünf dieser Systeme; oder L ist eine chemische Bindung;
- n: ist bei jedem Auftreten gleich oder verschieden 0 oder 1, wobei für n = 0 statt Y ein Wasserstoff oder Rest R¹ vorhanden ist, mit der Maßgabe, dass pro Einheit mindestens ein Index n ungleich 0 ist;
- p: ist 2, 3, 4, 5 oder 6, mit der Maßgabe, dass p nicht größer ist als die maximale Valenz von L;
dadurch gekennzeichnet, dass mindestens ein Rest R für eine Gruppe der folgenden Formel (3) steht, 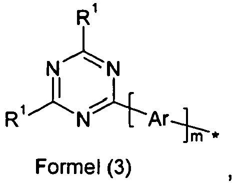 und/oder dass mindestens eine Gruppe L für eine Gruppe der folgenden Formeln (10) oder (11) steht, 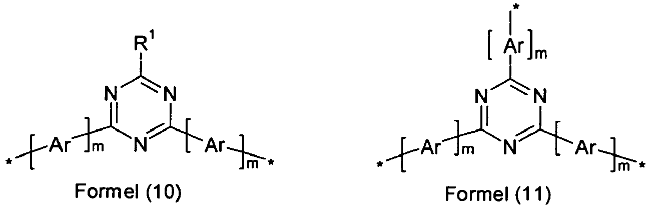 wobei R¹ für ein aromatisches oder heteroaromatisches Ringsystem mit 5 bis 20 aromatischen Ringatomen steht, das keine kondensierten Aromaten mit mehr als 10 aromatischen Ringatomen aufweist und welches jeweils durch einen oder mehrere Reste R² substituiert sein kann, und die weiteren verwendeten Symbole die oben genannten Bedeutungen haben und der Index m für 0 oder 1 steht; * deutet dabei die Position der Bindung der Gruppe gemäß Formel (3), (10) bzw. (11) an.

Dabei kann die Gruppe der Formel (3) entweder an einen der Phenylringe der Verbindung gemäß Formel (17) oder (18) gebunden sein, oder sie kann an die Gruppe Y gebunden sein.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 80 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe unterbrochen sind.

Im Rahmen der vorliegenden Erfindung werden unter einem aliphatischen Kohlenwasserstoffrest bzw. einer Alkylgruppe bzw. einer Alkenyl- oder Alkinylgruppe, die typischerweise 1 bis 40 oder auch 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinyithio, Heptinyithio oder Octinyithio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 80 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, indolizin und Benzothiadiazol oder Gruppen, die abgeleitet sind von Kombination dieser Systeme.

Gruppen R, die in Y gebunden sind, sind bevorzugt gleich oder verschieden bei jedem Auftreten ausgewählt aus der Gruppe bestehend aus Alkylgruppen mit 1 bis 10 C-Atomen oder aromatischen oder heteroaromatischen Ringsystemen mit 5 bis 20 aromatischen Ringatomen, welche jeweils durch einen oder mehrere Reste R¹ substituiert sein können. In einer besonders bevorzugten Ausführungsform der Erfindung sind die Gruppen R, die an Y gebunden sind, gleich oder verschieden bei jedem Auftreten ausgewählt aus aromatischen oder heteroaromatischen Ringsystemen mit 5 bis 20 aromatischen Ringatomen, welche jeweils durch einen oder mehrere Reste R¹ substituiert sein können. In einer weiteren besonders bevorzugten Ausführungsform ist ein Rest R, wenn Y für C(R)₂ steht, eine Alkylgruppe mit 1 bis 10 C-Atomen und der andere an dieses Kohlenstoffatom gebundene Rest R ein aromatisches oder heteroaromatisches Ringsystem mit 5 bis 20 aromatischen Ringatomen, welches mit einem oder mehreren Resten R¹ substituiert sein kann.

Besonders bevorzugt sind die Verbindungen der folgenden Formeln (20), (21), (23) oder (24), 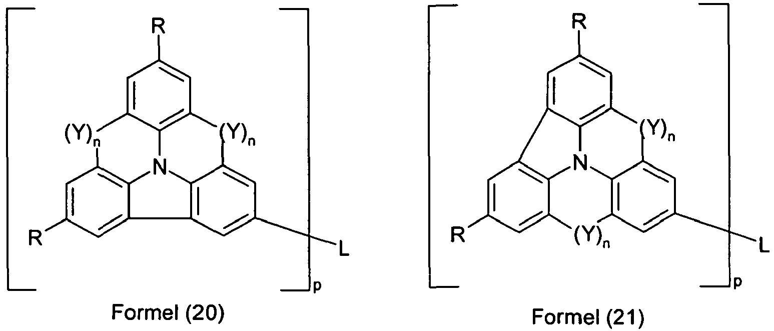 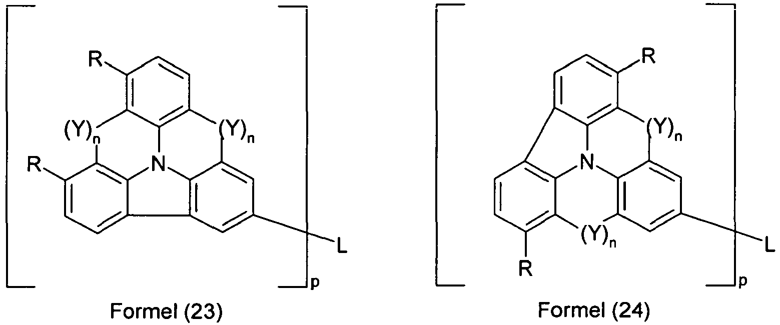 wobei die als nicht substituiert gezeichneten C-Atome auch durch D statt H substituiert sein können und die weiteren Symbole und Indizes die oben genannten Bedeutungen aufweisen. Dabei weisen die Reste R, die an Y gebunden sind, bevorzugt die oben genannten bevorzugten Bedeutungen auf.

In einer weiteren bevorzugten Ausführungsform der Erfindung ist L eine bivalente oder höher valente geradkettige Alkylengruppe mit 1 bis 10 C-Atomen oder eine verzweigte oder cyclische Alkylengruppe mit 3 bis 10 C-Atomen, die mit jeweils einem oder mehreren Resten R¹ substituiert sein kann, wobei ein oder mehrere H-Atome durch D oder F ersetzt sein können, oder ein mindestens bivalentes aromatisches oder heteroaromatisches Ringsystem mit 5 bis 24 aromatischen Ringatomen, welches durch einen oder mehrere Reste R¹ substituiert sein kann; oder L ist eine chemische Bindung; oder L ist eine Gruppe gemäß einer der Formeln (10) oder (11).

In einer bevorzugten Ausführungsform der Erfindung ist R gleich oder verschieden bei jedem Auftreten ausgewählt aus der Gruppe bestehend aus H, D, F, Cl, Br, CN, N(Ar)₂, C(=O)_{Ar}, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenyl- oder Alkinylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R¹ substituiert sein kann, einer Aryloxy- oder Heteroaryloxygruppe mit 5 bis 30 aromatischen Ringatomen, die mit einem oder mehreren Resten R¹ substituiert sein kann, oder einer Kombination dieser Systeme. Dabei ist wenigstens ein Rest R eine Gruppe der Formel (3), wie oben definiert.

In einer besonders bevorzugten Ausführungsform der Erfindung ist R gleich oder verschieden bei jedem Auftreten ausgewählt aus der Gruppe bestehend aus H, D, F, Cl, Br, CN, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R¹ substituiert sein kann, wobei ein oder mehrere H-Atome durch D oder F ersetzt sein können, einem aromatischen oder heteroaromatischen Ringsystem mit 5 bis 18 aromatischen Ringatomen, das jeweils mit einem oder mehreren Resten R¹ substituiert sein kann, oder einer Kombination dieser Systeme. Dabei ist, wie oben beschrieben, mindestens einer der Substituenten R ausgewählt aus Gruppen der Formel (3).

In einer weiteren bevorzugten Ausführungsform der Erfindung stehen die Symbole R, die nicht für eine Gruppe der Formeln (3) stehen und die nicht an Y binden, in Verbindungen der Formel (17) und (18) für H oder D.

Dabei haben für Verbindungen, die durch Vakuumverdampfung verarbeitet werden, die Alkylgruppen bevorzugt nicht mehr als vier C-Atome, besonders bevorzugt nicht mehr als 1 C-Atom. Für Verbindungen, die aus Lösung verarbeitet werden, eignen sich auch Verbindungen, die mit Alkylgruppen mit bis zu 10 C-Atomen substituiert sind oder die mit Oligoarylengruppen, beispielsweise ortho-, meta-, para- oder verzweigten Terphenylgruppen oder Quaterphenylgruppen, substituiert sind.

In einer weiteren bevorzugten Ausführungsform der Erfindung ist der Index p = 2 oder 3, besonders bevorzugt 2.

Wie oben beschrieben, steht mindestens einer der Reste R für eine Gruppe der oben genannten Formel (3) oder L steht für eine Gruppe der Formeln (10) oder (11). Dabei kann diese Gruppe R entweder an einen der Phenylringe des Grundgerüsts binden, oder sie kann an die Gruppe Y binden. In einer bevorzugten Ausführungsform der Erfindung bindet die Gruppe R der Formel (3) an einen der Phenylringe des Grundgerüsts.

In einer weiteren bevorzugten Ausführungsform stehen ein oder zwei Gruppen R für eine Gruppe der Formel (3), besonders bevorzugt genau eine Gruppe R.

Besonders bevorzugt sind daher die Verbindungen der folgenden Formeln (41), (42), (44), (45), (47) bis (50), (52), (53), (55), (57) und (58), 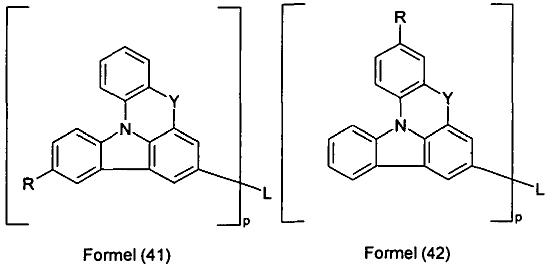 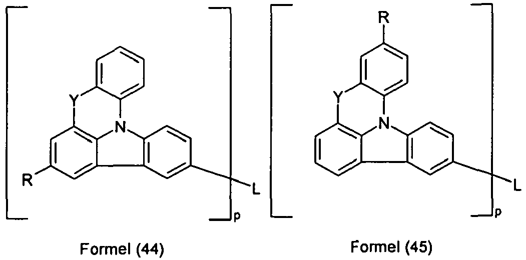 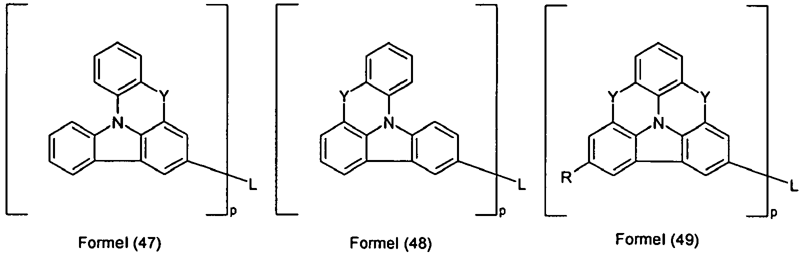 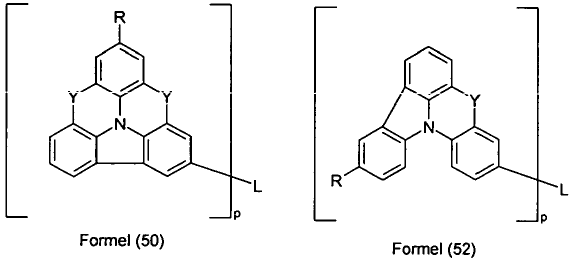 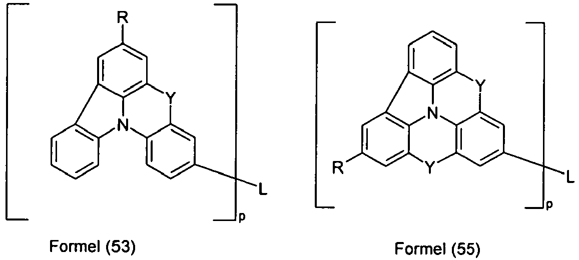 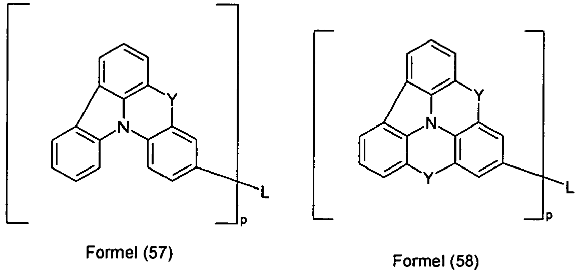 wobei R eine Gruppe gemäß einer der Formel (3) darstellt und wobei L in Formel (47), (48), (57) oder (58) eine Gruppe gemäß einer der Formeln (10) oder (11) darstellt, Y gleich oder verschieden, bevorzugt gleich bei jedem Auftreten für C(R)₂ steht, wobei R, welches in der C(R)₂-Gruppe gebunden ist, gleich oder verschieden bei jedem Auftreten für eine Alkylgruppe mit 1 bis 10 C-Atomen oder ein aromatisches oder heteroaromatisches Ringsystem mit 5 bis 18 aromatischen Ringatomen, welches jeweils durch einen oder mehrere Reste R¹ substituiert sein kann, steht; weiterhin können die als nicht substituiert gezeichneten C-Atome auch durch D statt H substituiert sein, und die weiteren Symbole und Indizes weisen die oben genannten Bedeutungen auf. Insbesondere steht R, welches in der C(R)₂-Gruppe gebunden ist, bevorzugt für die oben genannten bevorzugten Gruppen.

In einer weiteren bevorzugten Ausführungsform der Erfindung weisen die Strukturen der Formein (17), (18), (20), (21), (23), (24), (41), (42), (44), (45), (47) bis (50), (52), (53), (55), (57) und (58) jeweils in der Position para zum zentralen Atom X, also para zum Stickstoff, einen Rest R ungleich H oder D auf. Besonders bevorzugt stehen die Substituenten R in der para-Position von X, die nicht für eine Gruppe der Formel (3) stehen, für eine Alkylgruppe mit 1 bis 10 C-Atomen, insbesondere mit 1 bis 4 C-Atomen, oder für ein aromatisches oder heteroaromatisches Ringsystem mit 5 bis 24 aromatischen Ringatomen, welches durch einen oder mehrere Reste R¹ substiutiert sein kann, insbesondere für eine Phenylgruppe, welche durch einen oder mehrere Reste R¹ substituiert sein kann. Durch diese Bevorzugung sind die erfindungsgemäßen Verbindungen selektiver synthetisch zugänglich.

In den Strukturen der Formeln (3), (10) und (11) steht das Symbol R¹ bevorzugt für ein aromatisches oder heteroaromatisches Ringsystem mit 5 bis 20 aromatischen Ringatomen, das keine kondensierten Aromaten mit mehr als 10 aromatischen Ringatomen aufweist und welches jeweils durch einen oder mehrere Reste R² substituiert sein kann, besonders bevorzugt für Phenyl, ortho-, meta- oder para-Biphenyl, ortho-, meta- oder para-Terphenyl, Quaterphenyl oder 1- oder 2-Naphthyl, welches jeweils durch einen oder mehrere Reste R² substituiert sein kann.

In einer weiteren bevorzugten Ausführungsform der Erfindung steht die Gruppe Ar in den Formeln (3), (10) und (11) für ein aromatisches oder heteroaromatisches Ringsystem mit 5 bis 20 aromatischen Ringatomen, das keine kondensierten Aromaten mit mehr als 10 aromatischen Ringatomen aufweist und welches mit einem oder mehreren nicht-aromatischen Resten R¹ substituiert sein kann. Besonders bevorzugt steht Ar in den Formeln (3), (10) und (11) für Phenyl, ortho-, meta- oder para-Biphenyl, ortho-, meta- oder para-Terphenyl oder Quaterphenyl, welches jeweils mit einem oder mehreren nicht-aromatischen Resten R¹ substituiert sein kann, bevorzugt jedoch unsubstituiert ist.

Beispiele für bevorzugte Verbindungen gemäß den oben aufgeführten Ausführungsformen bzw. Verbindungen, wie sie bevorzugt in organischen elektronischen Vorrichtungen eingesetzt werden können, sind die Verbindungen der folgenden Strukturen.

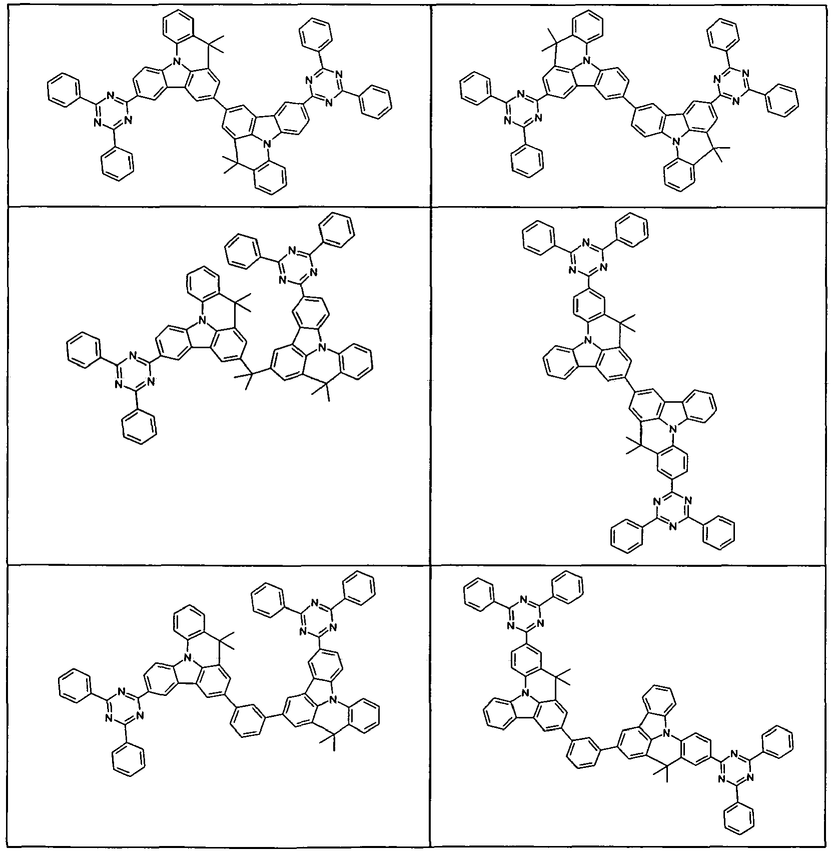

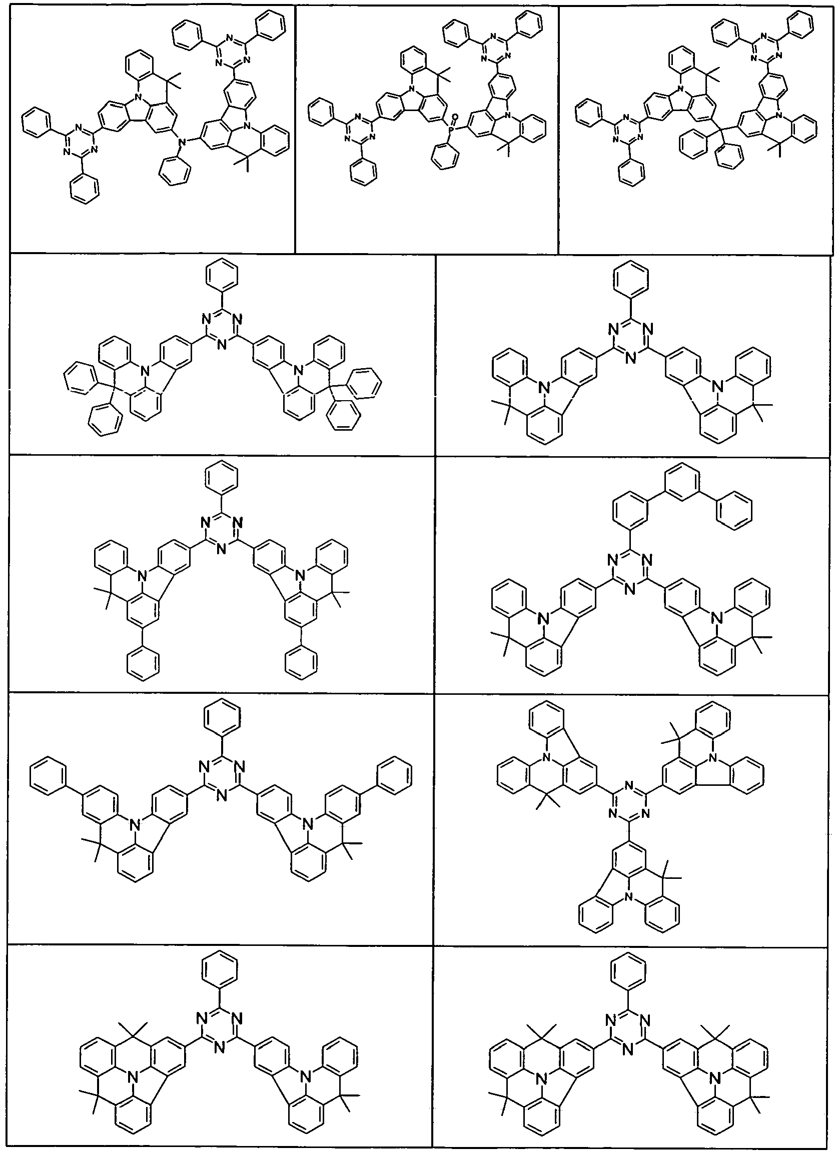

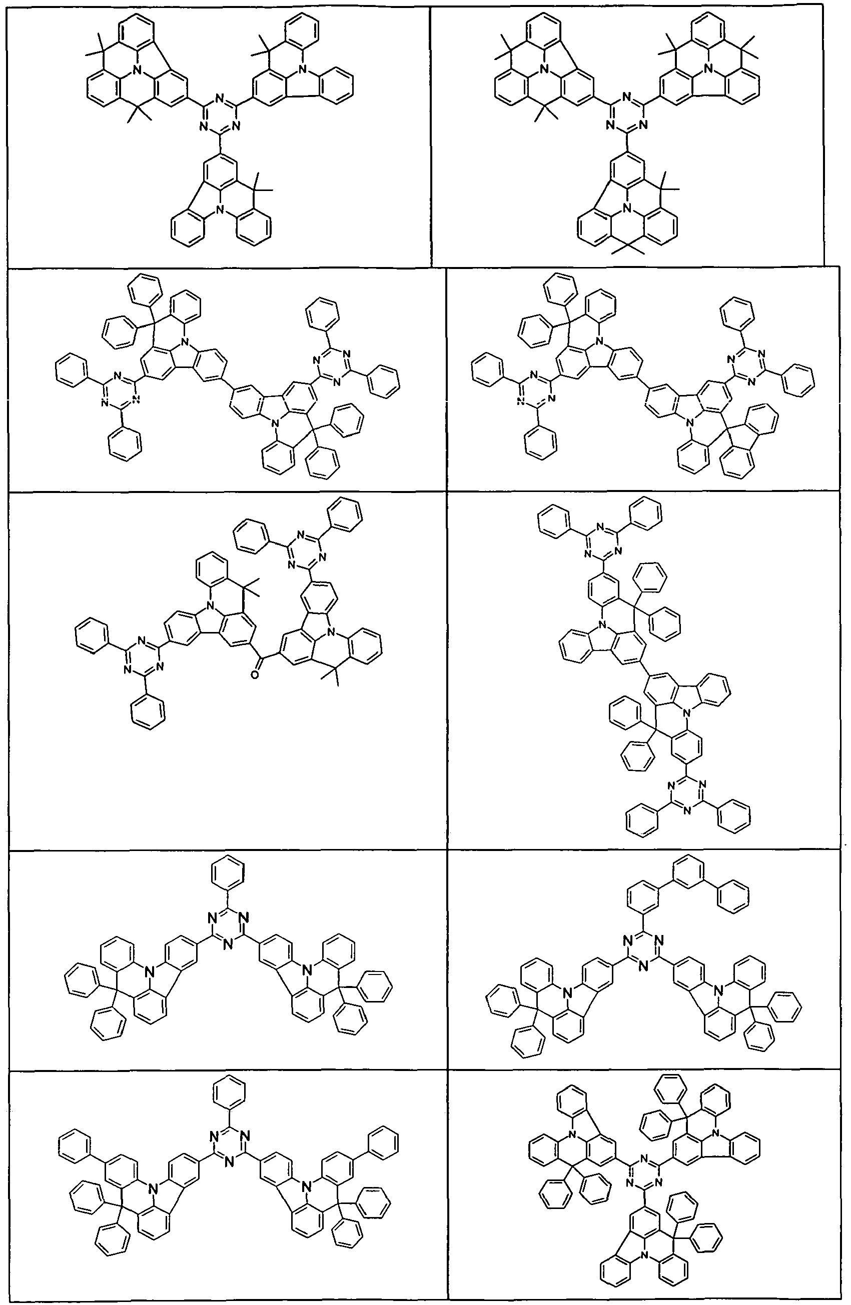

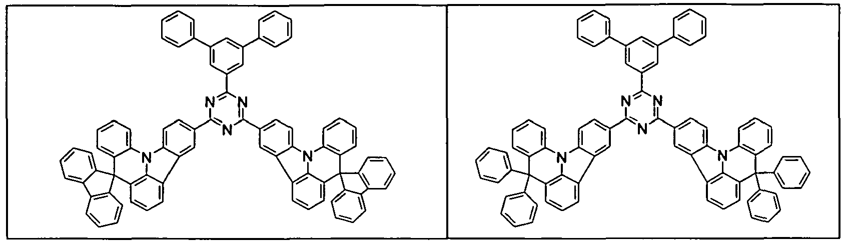

Die Grundgerüste der erfindungsgemäßen Verbindungen können nach dem Fachmann bekannten Syntheseschritten, wie z. B. Bromierung, Ullmann-Arylierung, Hartwig-Buchwald-Kupplung, etc., dargestellt werden, wie in Schema 1 bis 3 dargestellt. Diese Grundgerüste können in einem weiteren Schritt funktionalisiert werden. So führt die Bromierung von Carbazolderivaten, welche eine oder zwei Brücken Y aufweisen, zu mono- oder di-p-Brom-substituierten verbrückten Carbazolderivaten. Als Bromierungsagens können neben elementarem Brom vor allem auch N-Brom-Verbindungen wie N-Bromsuccinimid (NBS) verwendet werden. Ebenso geeignet ist die Funktionalisierung mit anderen reaktiven Gruppen, beispielsweise Chlor, Iod, Boronsäure bzw. Boronsäurederivaten, insbesondere Boronsäureester, Triflat oder Tosylat.

Je nach gewünschter Bromsubstitution kann eine Cyclisierung über die Zwischenstufe eines tertiären Alkohols vor (Schema 1) oder nach der Bromierung (Schema 2) des Carbazols erfolgen. Durch den Ringschluss entsteht eine bivalente Brücke zwischen dem aromatischen Substituenten und dem Carbazol (siehe Schema 1-3). Dabei eignet sich beispielsweise eine Carbonsäureestergruppe oder eine Acetylgruppe, welche dann in der Ringschlussreaktion zu einer Kohlenstoffbrücke umgesetzt werden kann (Schema 1 und 2). Dabei steht R in den Schemata für einen Substituenten, wie oben definiert.

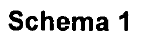

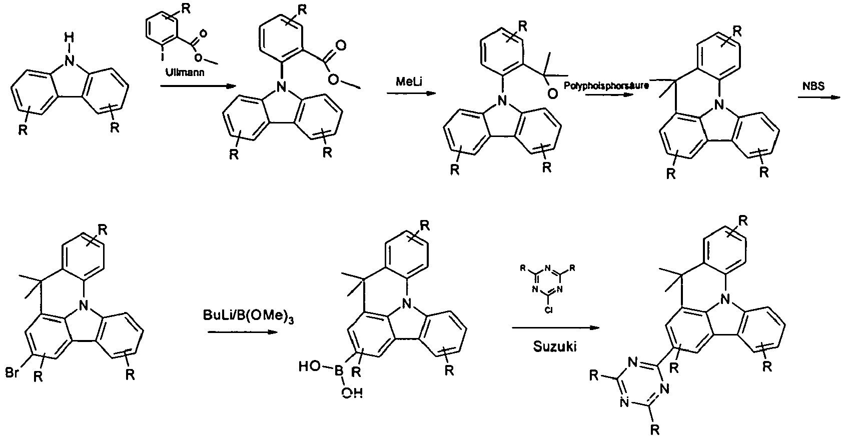

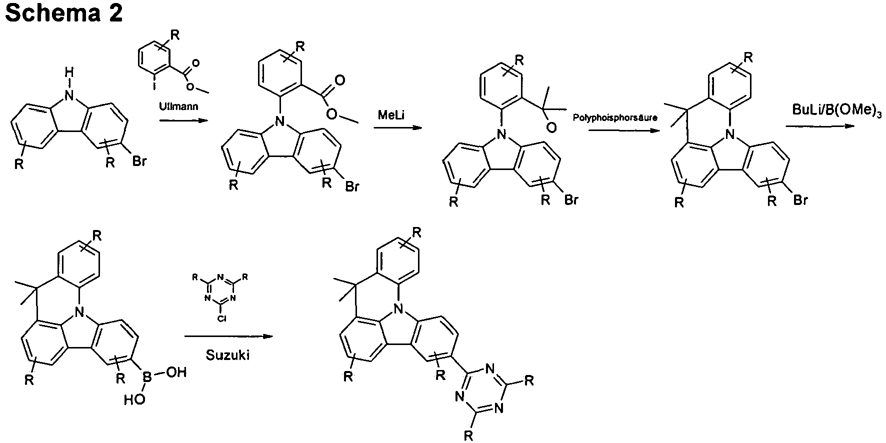

Die Einführung aromatischer Substituenten an der Brücke Y ist exemplarisch im folgenden Schema 3 gezeigt. Dabei wird der Carbonsäureester statt mit einer Alkyl-organischen Verbindung mit einer Aryl-organischen Verbindung, beispielsweise einer aromatischen Grignard-Verbindung, umgesetzt.

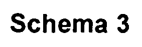

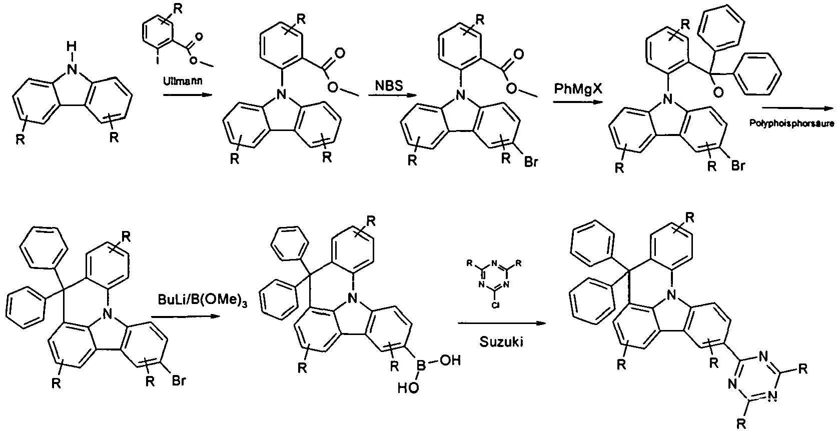

Die bivalente Brücke kann im weiteren Verlauf mit weiteren Resten substituiert werden, beispielsweise mit Alkyl- oder Arylgruppen. Die so hergestellte verbrückte Carbazolverbindung kann nun in einem weiteren Schritt funktionalisiert werden, beispielsweise halogeniert, bevorzugt bromiert.

Die funktionalisierten, insbesondere bromierten Verbindungen stellen den zentralen Baustein für die weitere Funktionalisierung dar, wie in Schema 1 bis 3 dargestellt. So lassen sich diese funktionalisierten verbrückten Verbindungen leicht in entsprechende Boronsäuren überführen und beispielsweise durch Suzuki-Kupplung mit 2-Chlor-4,6-diphenyl-1,3,5-triazin oder anderen Chlortriazinderivaten in Verbindungen gemäß Formel (17) oder (18) umsetzen.

Ebenso können andere Kupplungsreaktionen (z. B. Stille-Kupplung, Heck-Kupplung, Sonogashira-Kupplung, etc.) Verwendung finden. Kupplung mit Diarylaminen nach Hartwig-Buchwald führt zu Triarylamin-Derivaten. Entsprechend können aliphatische Amine, Carbazole, etc. als Substituenten eingeführt werden. Als Funktionalisierung kommen weiterhin Formyl-, Alkylcarbonyl- und Arylcarbonyl-Gruppen oder deren geschützte Analoga, z. B. in Form der entsprechenden Dioxolane, in Frage. Die bromierten Verbindungen können weiterhin lithiiert und durch Reaktion mit Elektrophilen wie Benzonitril und anschließender saurer Hydrolyse zu Ketonen oder mit Chlordiphenylphosphinen und anschließender Oxidation zu Phosphinoxiden umgesetzt werden.

Die Verbindungen gemäß Formel (17) oder (18) können somit durch eine Reaktionsfolge synthetisiert werden, welche die folgenden Reaktionsschritte umfasst:
a) Synthese des Grundgerüsts, welches statt der Gruppe R eine reaktive Abgangsgruppe trägt; und
b) Einführung der Gruppe R, bevorzugt durch eine Kupplungsreaktion, beispielsweise Suzuki-Kupplung oder Hartwig-Buchwald-Kupplung.

Dabei ist die reaktive Abgangsgruppe bevorzugt ausgewählt aus Cl, Br, I, Boronsäure bzw. Boronsäurederivaten, Triflat oder Tosylat.

Ein weiterer Gegenstand der vorliegenden Erfindung ist die Verwendung einer Verbindung der Formel (17) oder (18) oder der bevorzugten Ausführungsformen als Matrixmaterial für phosphoreszierende Emitter in einer organischen Elektrolumineszenzvorrichtung.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 05/011013). Ebenso ist es möglich, mehrere Emitter in eine emittierende Schicht zu dotieren und so aus einer Schicht weiße Emission zu erzeugen.

Dabei kann die organische Elektrolumineszenzvorrichtung eine emittierende Schicht enthalten, oder sie kann mehrere emittierende Schichten enthalten, wobei mindestens eine emittierende Schicht mindestens eine Verbindung der Formel (17) oder (18) ais Matrixmaterial enthält.

Die Verbindung gemäß Formel (17) oder (18) bzw. gemäß den bevorzugten Ausführungsformen wird als Matrixmaterial für eine emittierende Verbindung in einer emittierenden Schicht eingesetzt wird in Kombination mit einem oder mehreren phosphoreszierenden Materialien (Triplettemitter). Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen, insbesondere alle Iridium-, Platin- und Kupferkomplexe als phosphoreszierende Verbindungen angesehen werden.

Die Mischung aus der Verbindung gemäß Formel (17) oder (18) bzw. den bevorzugten Ausführungsformen und der emittierenden Verbindung enthält zwischen 99.9 und 1 Vol.-%, vorzugsweise zwischen 99 und 10 Vol.-%, besonders bevorzugt zwischen 97 und 60 Vol.-%, insbesondere zwischen 95 und 80 Vol.-% der Verbindung gemäß Formel (17) oder (18) bzw. den bevorzugten Ausführungsformen bezogen auf die Gesamtmischung aus Emitter und Matrixmaterial. Entsprechend enthält die Mischung zwischen 0.1 und 99 Vol.-%, vorzugsweise zwischen 1 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der Verbindung gemäß Formel (17) oder (18) bzw. den bevorzugten Ausführungsformen als Matrixmaterial für einen phosphoreszierenden Emitter in Kombination mit einem weiteren Matrixmaterial. Besonders geeignete Matrixmaterialien, welche in Kombination mit den Verbindungen gemäß Formel (17) oder (18) bzw. den bevorzugten Ausführungsformen eingesetzt werden können, sind aromatische Ketone, aromatische Phosphinoxide oder aromatische Sulfoxide oder Sulfone, z. B. gemäß WO 04/013080, WO 04/093207, WO 06/005627 oder WO 10/006680, Triarylamine, Carbazolderivate, z. B. CBP (N,N-Biscarbazolylbiphenyl) oder die in WO 05/039246, US 2005/0069729, JP 2004/288381, EP 1205527 oder WO 08/086851 offenbarten Carbazolderivate, Indolocarbazolderivate, z. B. gemäß WO 07/063754 oder WO 08/056746, Indenocarbazolderivate, z. B. gemäß den nicht offen gelegten Anmeldungen DE 102009023155.2 oder DE 102009031021.5, Azacarbazolderivate, z. B. gemäß EP 1617710, EP 1617711, EP 1731584, JP 2005/347160, bipolare Matrixmaterialien, z. B. gemäß WO 07/137725, Silane, z. B. gemäß WO 05/111172, Azaborole oder Boronester, z. B. gemäß WO 06/117052, Triazinderivate, z. B. gemäß WO 10/015306, WO 07/063754 oder WO 08/056746, Zinkkomplexe, z. B. gemäß EP 652273 oder WO 09/062578, Diazasilol- bzw. Tetraazasilol-Derivate, z. B. gemäß WO 10/054729, oder Diazaphosphol-Derivate, z. B. gemäß WO 10/054730. Ebenso kann ein weiterer phosphoreszierender Emitter, welcher kürzerwellig als der eigentliche Emitter emittiert, als Co-Host in der Mischung vorhanden sein.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für geeignete phosphoreszierende Verbindungen sind in der folgenden Tabelle aufgeführt.

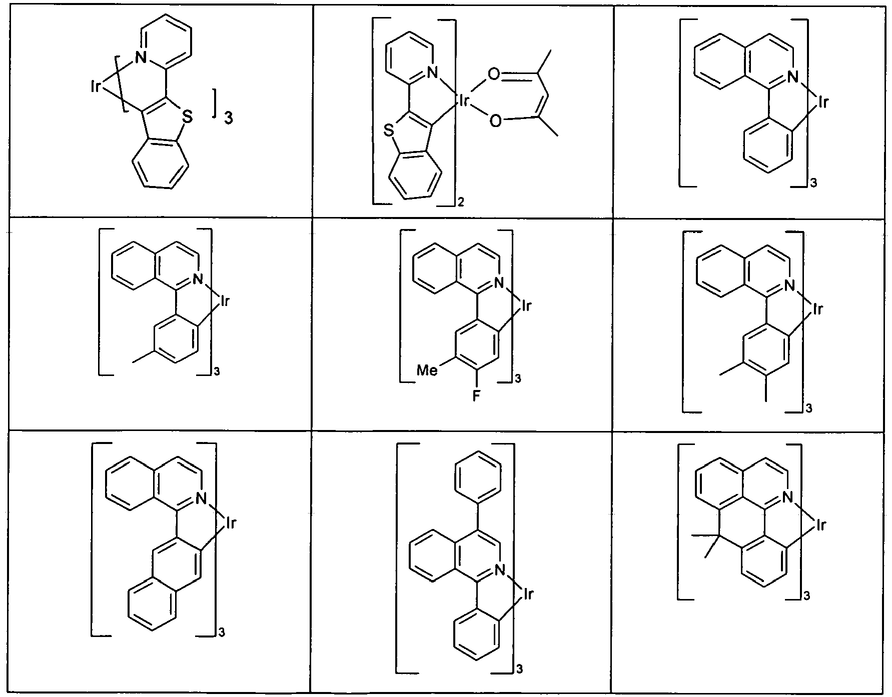

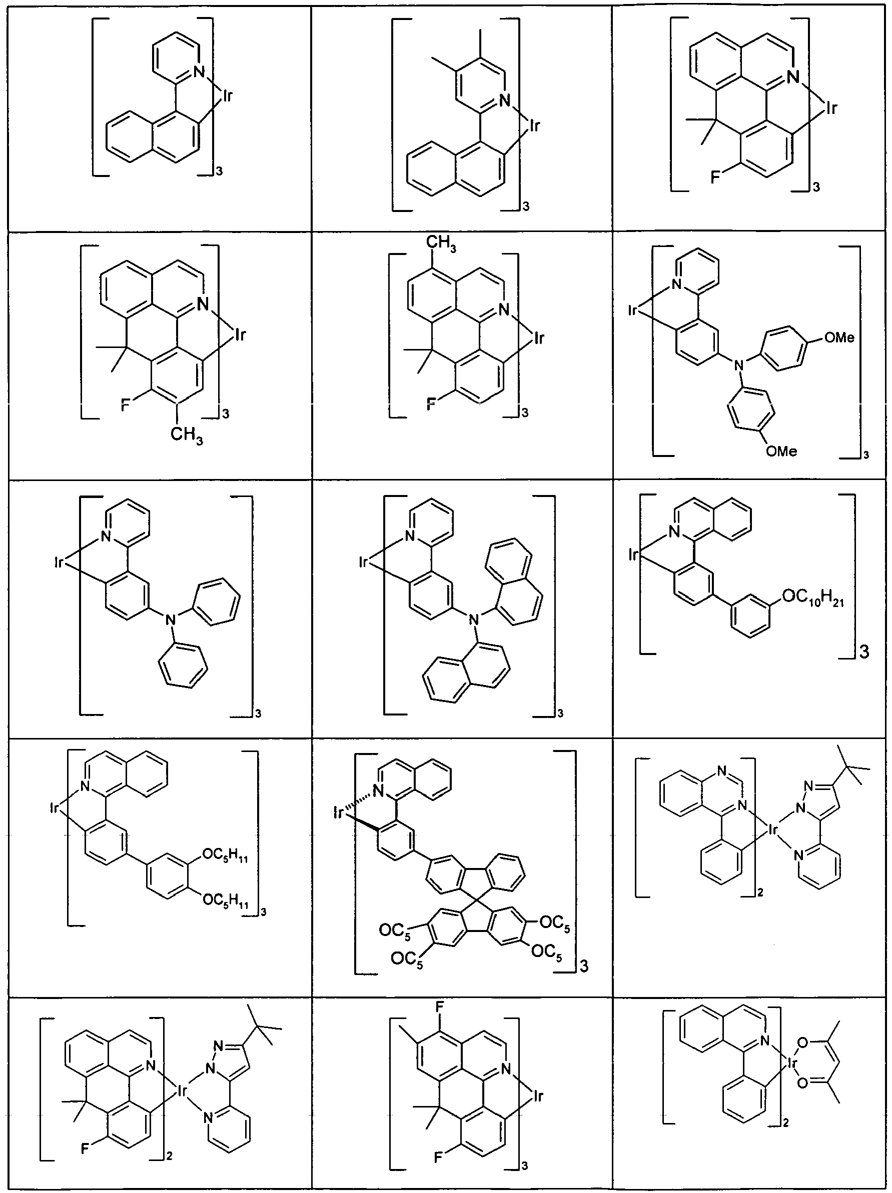

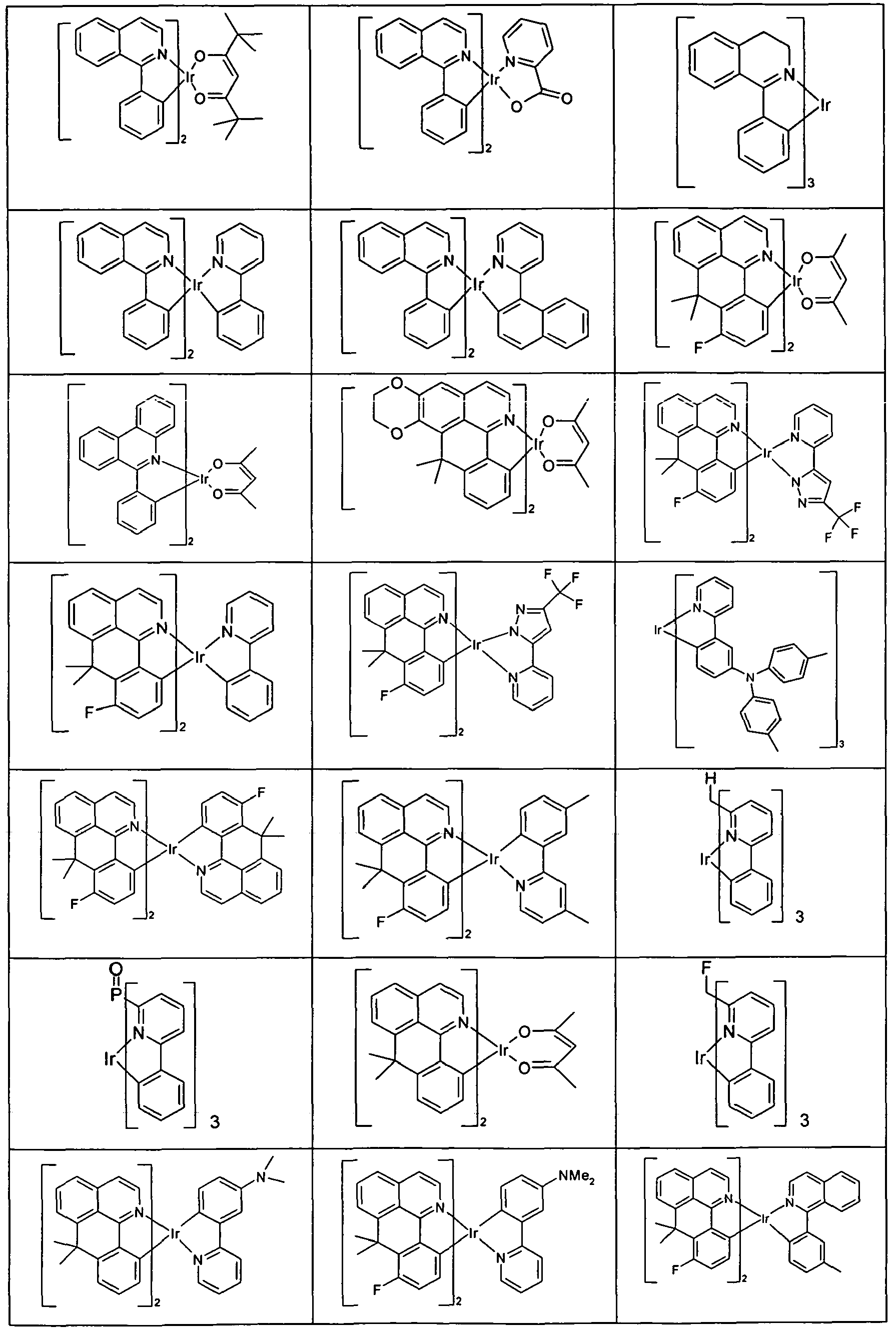

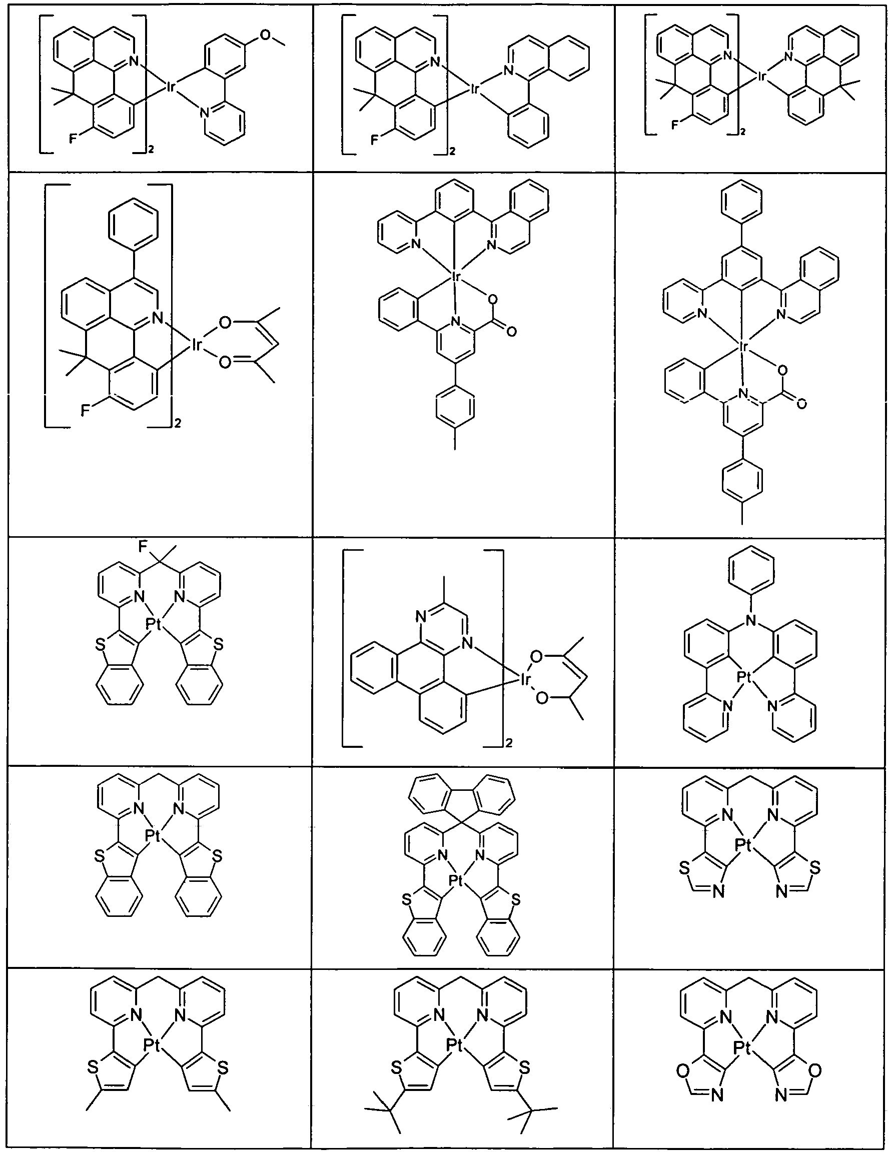

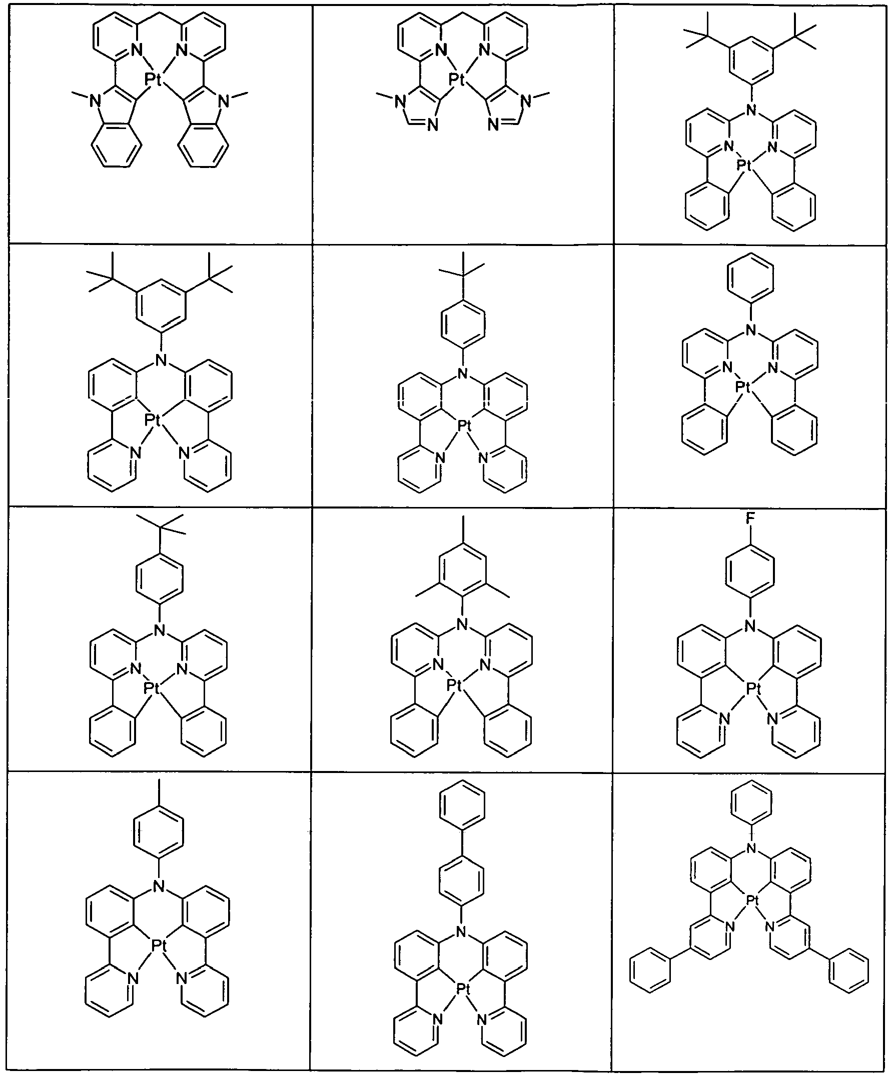

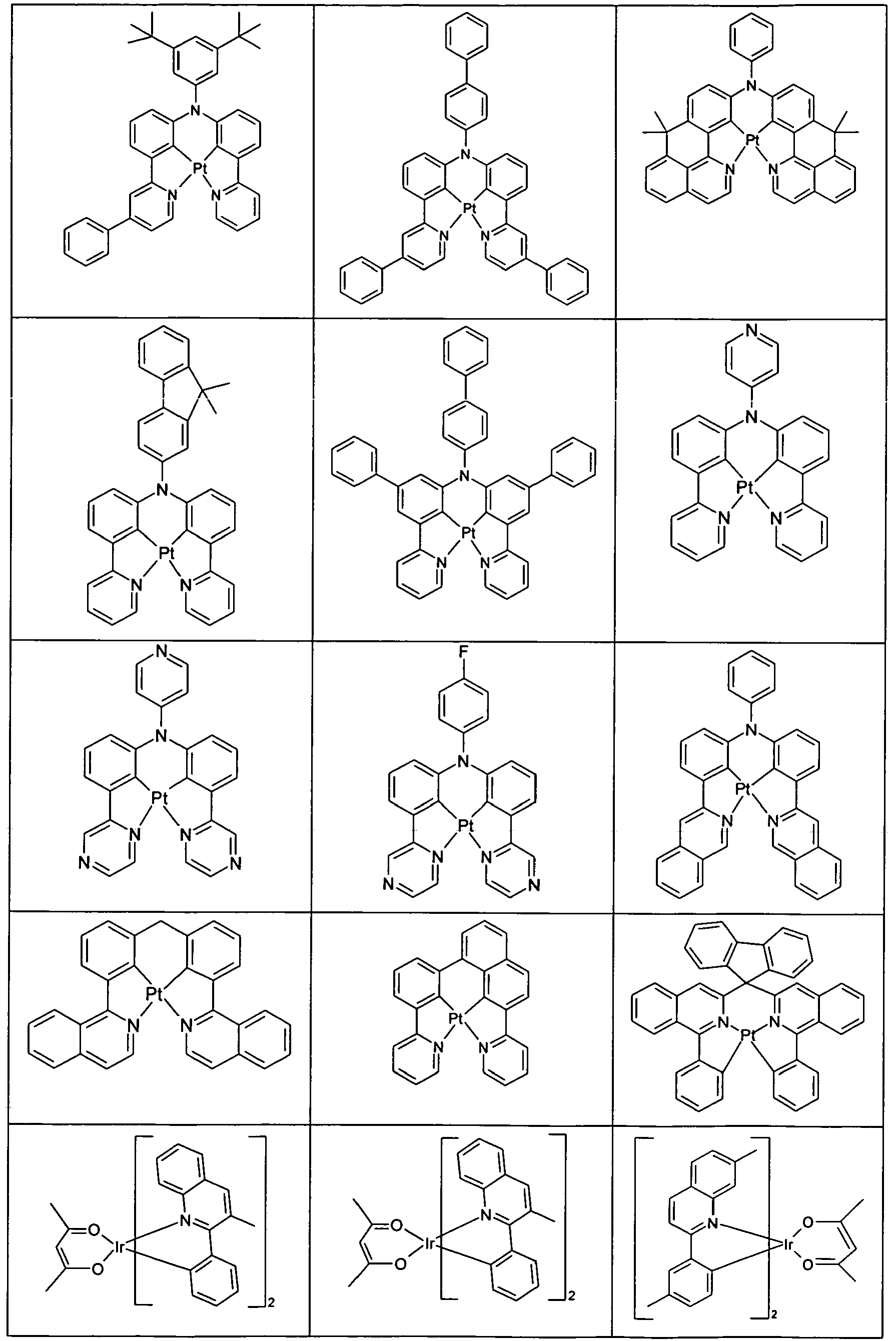

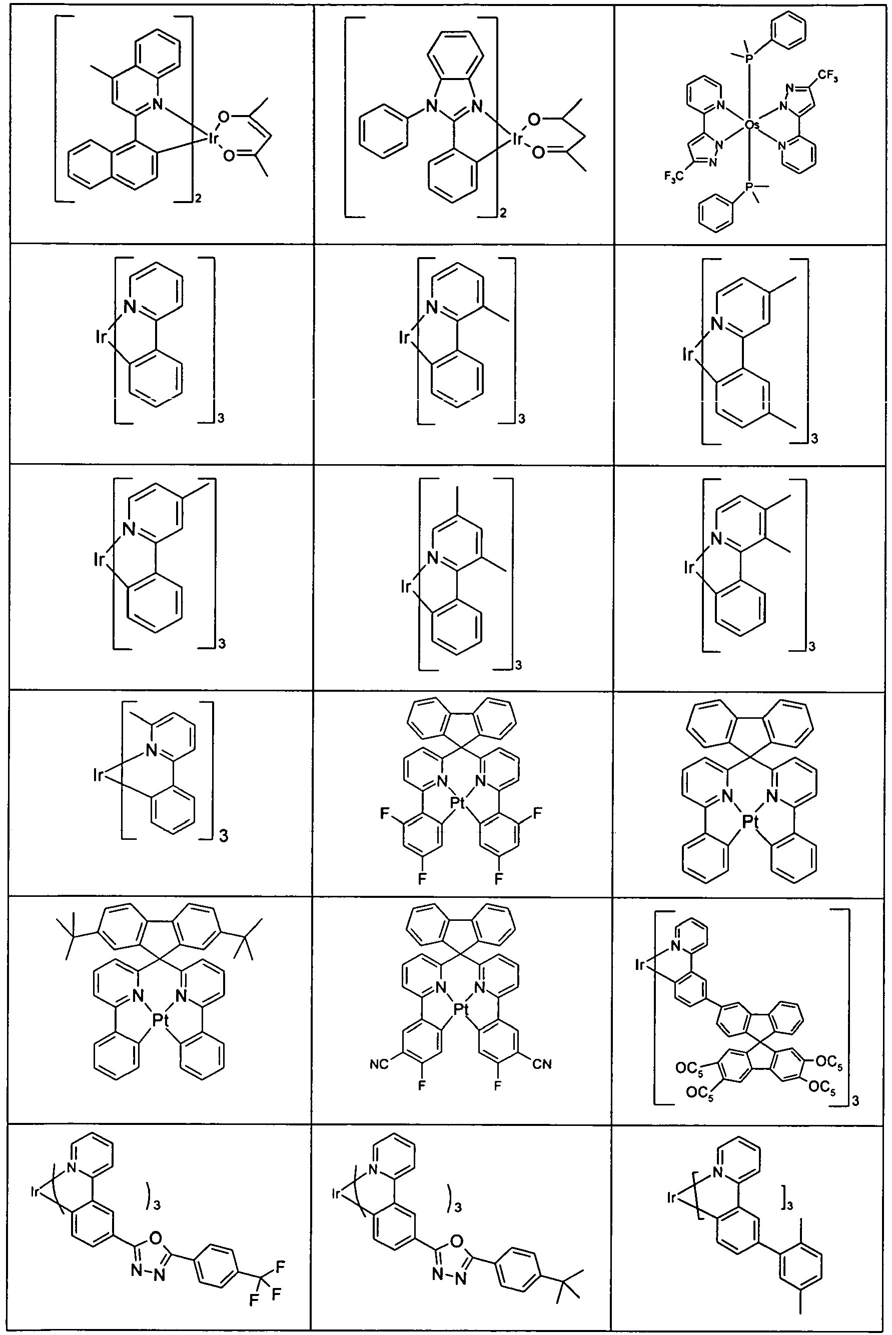

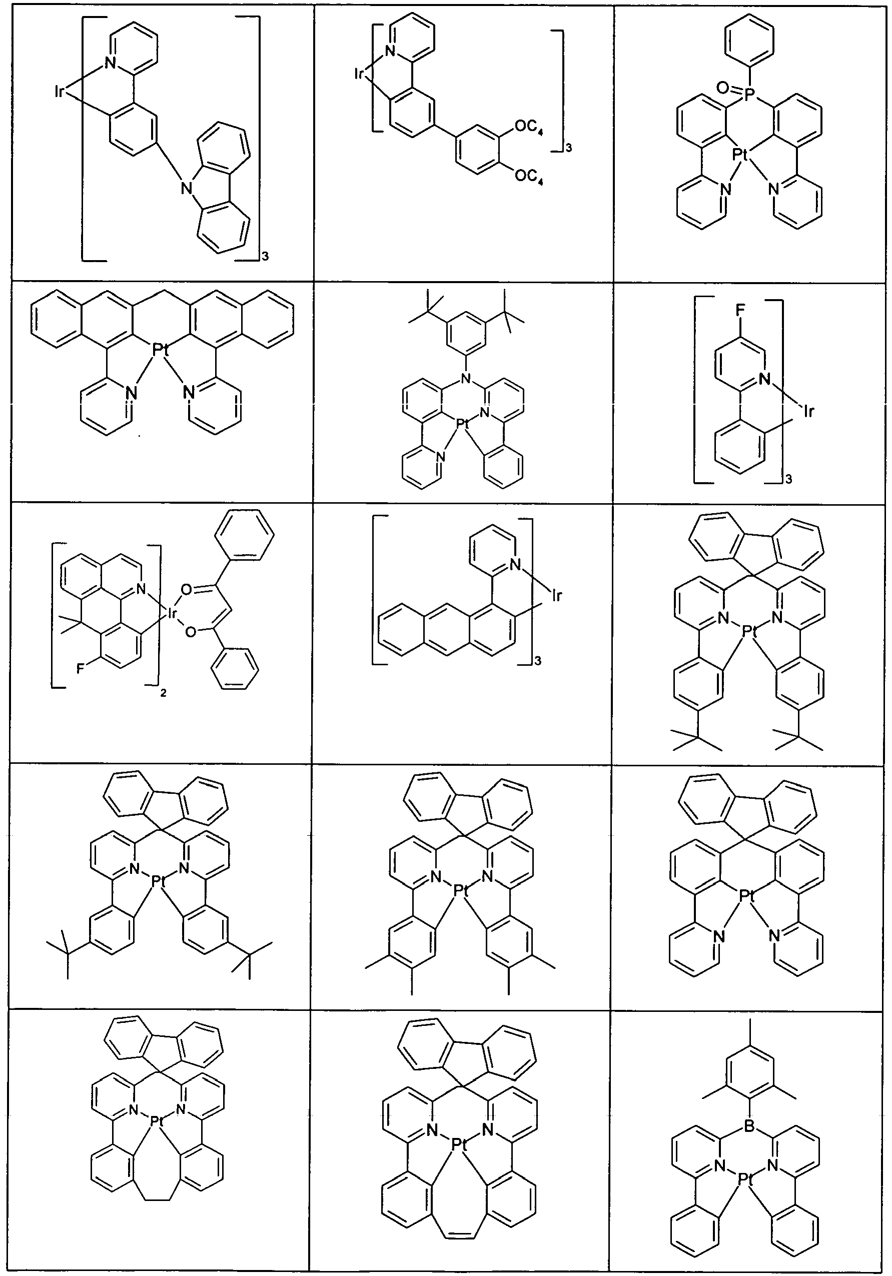

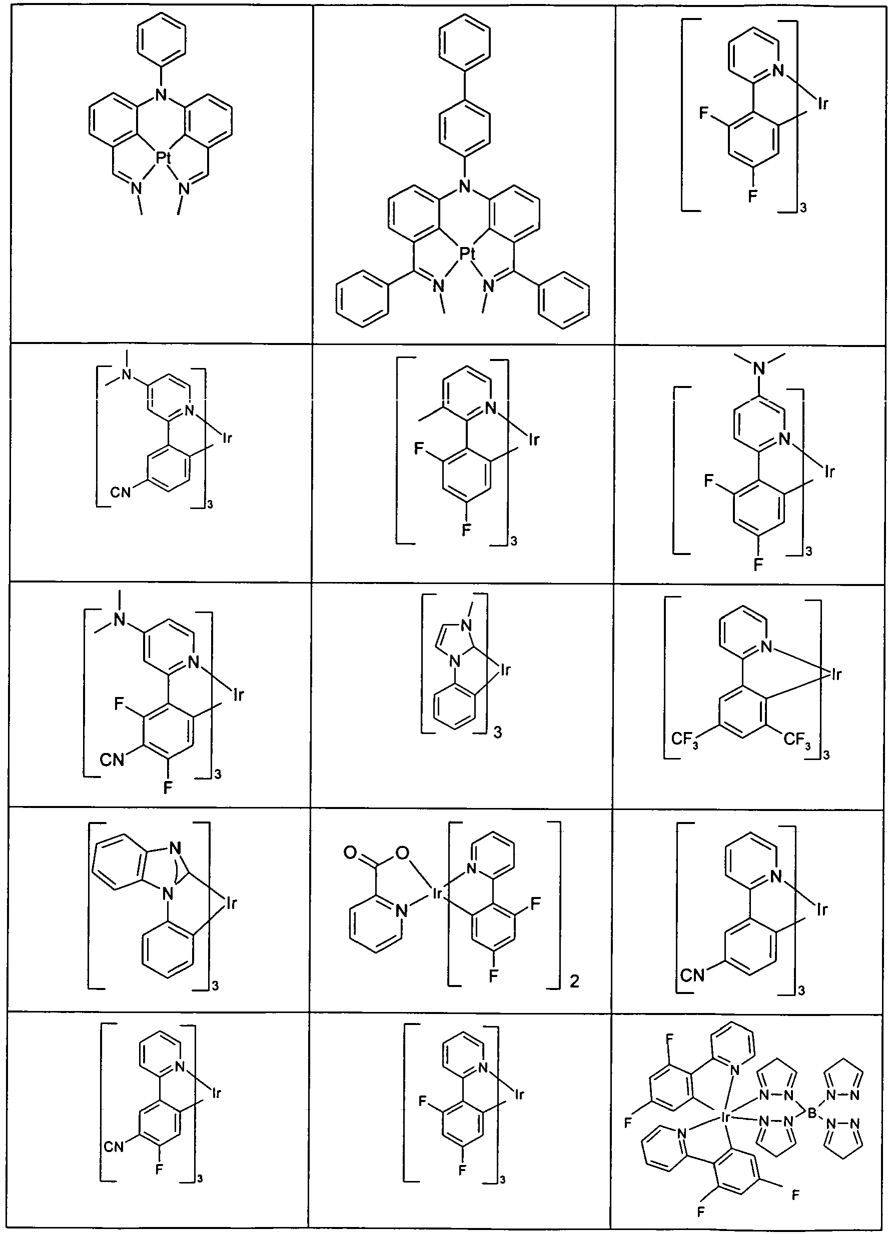

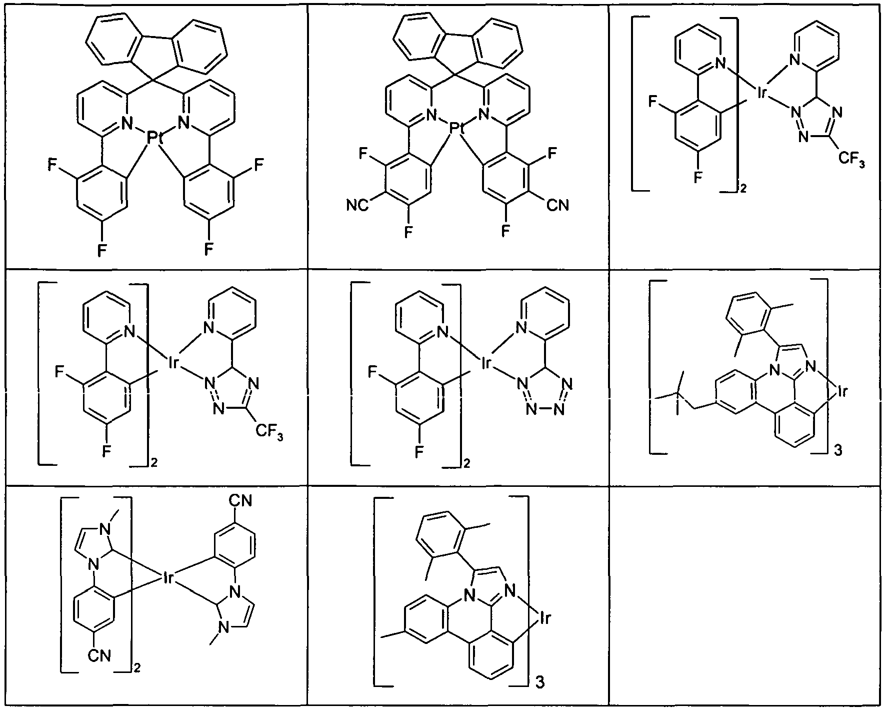

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 05/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 09/030981 beschrieben.

Es ist weiterhin möglich, die Verbindung gemäß Formel (17) oder (18) bzw. gemäß den bevorzugten Ausführungsformen sowohl in einer Lochblockierschicht bzw. Elektronentransportschicht als auch als Matrix in einer emittierenden Schicht zu verwenden.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den Verbindungen gemäß Formel (17) oder (18) bzw. den bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen organischen Elektrolumineszenzvorrichtungen zeichnen sich durch folgende überraschende Vorteile gegenüber dem Stand der Technik aus:
1. Die Verbindungen gemäß Formel (17) oder (18) bzw. den bevorzugten Ausführungsformen, eingesetzt als Matrixmaterial für phosphoreszierende Emitter führen zu sehr hohen Effizienzen sowie zu langen Lebensdauern.
2. Die erfindungsgemäßen Verbindungen bzw. Verbindungen gemäß Formel (17) oder (18) bzw. den bevorzugten Ausführungsformen eignen sich nicht nur als Matrix für rot phosphoreszierende Verbindungen, sondern insbesondere auch für grün phosphoreszierende Verbindungen.
3. Die Verbindungen gemäß Formel (17) und (18) führen zu hohen Effizienzen und zu steilen Strom-Spannungs-Kurven mit niedrigen Einsatzspannungen.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre durchgeführt. Die Edukte können von den Firmen ALDRICH bzw. ABCR (Palladium(II)acetat, Tri-o-tolylphosphin, Anorganika, Lösemittel) bezogen werden. Die Synthese von 8,8-Dimethyl-indolo[3,2,1-de]acridin und 7,7,11,11-Tetramethyl-7H,11H-benz[1,8]indoio-[2,3,4,5,6-de]acridin kann gemäß der Literatur (Chemische Berichte 1980, 113, 1, 358-84) erfolgen. Ebenso ist die Synthese von 8H-Indolo[3,2,1-de]phenazin (Journal of the Chemical Society 1958, 4492-4) und B-[4-(1-Phenyl-1 H-benzimidazol-2-yl)phenyl]boronsäure (Advanced Functional Materials 2008, 18, 4, 584-590) aus der Literatur bekannt.

### Beispiel 1: 6-Brom-8,8-dimethyl-8H-indolo[3,2,1,-de]acridin

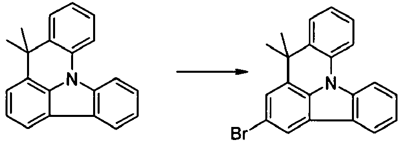

6.3 g (22.2 mmol) 8,8-Dimethyl-indolo[3,2,1-de]acridin werden in 150 mL CH₂Cl₂ vorgelegt. Anschließend tropft man unter Lichtausschluss bei -15 °C eine Lösung aus 8 g (45.1 mmol) NBS in 100 ml Acetonitril zu, lässt auf Raumtemperatur kommen und rührt 4 h weiter bei dieser Temperatur. Anschließend wird die Mischung mit 150 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt.
Ausbeute: 4.5 g (12 mmol), 57 % d. Th., Reinheit nach ¹H-NMR ca. 97 %.

### Beispiel 2: 2,5-Dibrom-7,7,11,11-tetramethyl-7H,11H-benz[1,8]indolo-[2,3,4,5,6-de]acridin

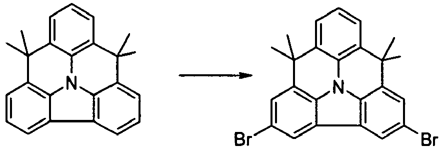

7.18 g (22.2 mmol) 7,7,11,11-Tetramethyl-7H,11H-benz[1,8]indolo-[2,3,4,5,6-de]acridin werden in 150 mL CH₂Cl₂ vorgelegt. Anschließend tropft man unter Lichtausschluss bei 0 °C eine Lösung aus 8 g (45.1 mmol) NBS in 100 ml CH₂Cl₂ hinzu, lässt auf Raumtemperatur kommen und rührt 4 h weiter bei dieser Temperatur. Anschließend wird die Mischung mit 150 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt.
Ausbeute: 8.1 g (16 mmol), 70 % d. Th., Reinheit nach ¹H-NMR ca. 98 %.

### Beispiel 3: 8,8-Dimethyl-8H-indolo[3,2,1-de]acridin-6-boronsäure

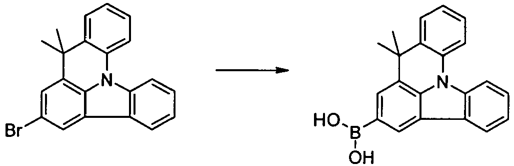

93.9 g (259 mmol) 6-Brom-8,8-dimethyl-8H-indolo[3,2,1,-de]acridin werden in 1500 mL trockenem THF gelöst, bei -70 °C 135 mL (337 mmol) einer 2.5 M Lösung von n-Butyllithium in Cyclohexan zugetropft, nach 1 h 37 mL Trimethylborat (336 mmol) zugetropft, innerhalb 1 h auf Raumtemperatur kommen gelassen, das Lösungsmittel entfernt und der Rückstand, der nach ¹H-NMR einheitlich ist, ohne weitere Reinigung in die Folgereaktion eingesetzt. Die Ausbeute beträgt 77 g (235 mmol), entsprechend 91 % der Theorie.

### Beispiel 4: 7,7,11,11-Tetramethyl-7H,11H-benz[1,8]indolo[2,3,4,5,6-de]acridin-2,5-bisboronsäure

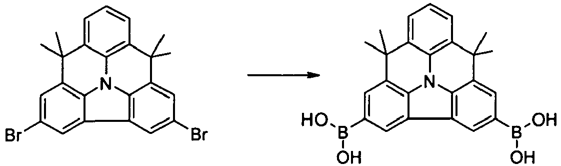

56.2 g (154 mmol) 2,5 Dibrom-7,7,11,11-tetramethyl-7H,11H-benz[1,8]-indolo[2,3,4,5,6-de]acridin werden in 1400 mL trockenem THF gelöst, bei -70 °C 162 mL (404 mmol) einer 2.5 M Lösung von n-Butyllithium in Cyclohexan zugetropft, nach 1 h 44.4 mL Trimethylborat (403 mmol) zugetropft, innerhalb 1 h auf RT kommen gelassen, das Lösungsmittel entfernt und der Rückstand, der nach ¹H-NMR einheitlich ist, ohne weitere Reinigung in die Folgereaktion eingesetzt. Die Ausbeute beträgt 33 g (80 mmol), entsprechend 69 % der Theorie.

### Beispiel 5: 6-(4,6-Diphenyl-[1,3,5]triazin-2-yl)-8,8-dimethyl-8H-indolo[3,2,1,-de]acridin (nicht Gegenstand der Erfindung)

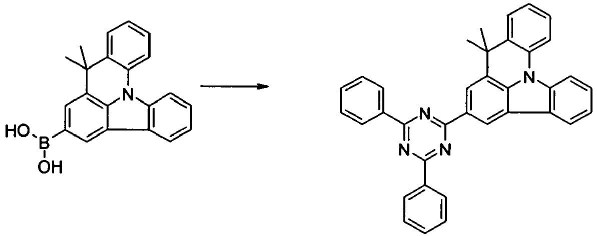

36 g (110.0 mmol) 8,8-Dimethyl-8H-indolo[3,2,1-de]acridin-6-boronsäure, 29.5 g (110.0 mmol) 2-Chlor-4,6-diphenyl-1,3,5-triazin und 44.6 g (210.0 mmol) Trikaliumphosphat werden in 500 mL Toulol, 500 mL Dioxan und 500 mL Wasser suspendiert. Zu dieser Suspension werden 913 mg (3.0 mmol) Tri-o-tolylphosphin und dann 112 mg (0.5 mmol) Palladium(II)-acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan / *iso*-Propanol umkristallisiert und abschließend im Hochvakuum sublimiert, Reinheit beträgt 99.9 %. Die Ausbeute beträgt 46 g (89 mmol), entsprechend 83 % der Theorie.

### Beispiel 6: 2,5-Bis(4,6-diphenyl-[1,3,5]triazin-2-yl)-7,7,11,11-tetramethyl-7H,11H-benz[1,8]indolo[2,3,4,5,6-de]acridin (nicht Gegenstand der Erfindung)

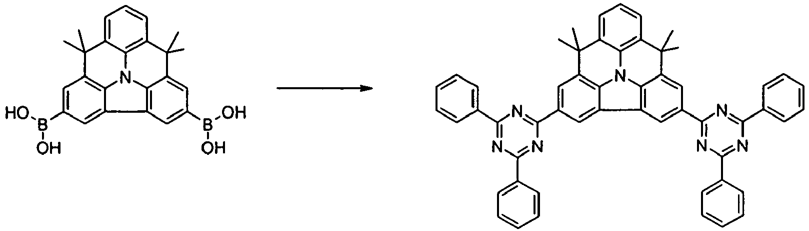

22.6 g (55 mmol) 7,7,11,11-Tetramethyl-7H,11H-benz[1,8]indolo-[2,3,4,5,6-de]acridin-2,5-bisboronsäure, 29.5 g (110.0 mmol) 2-Chlor-4,6-diphenyl-1,3,5-triazin und 44.6 g (210.0 mmol) Trikaliumphosphat werden in 500 mL Toulol, 500 mL Dioxan und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan / *iso*-Propanol umkristallisiert und abschließend im Hochvakuum sublimiert, Reinheit beträgt 99.9 %. Die Ausbeute beträgt 30 g (38 mmol), entsprechend 72 % der Theorie.

### Beispiel 7: [6-(1-Phenyl-1H-benzoimidazol-2-yl)-phenyl]-8,8-dimethyl-8H-indolo[3,2,1,-de]acridin (nicht Gegenstand der Erfindung)

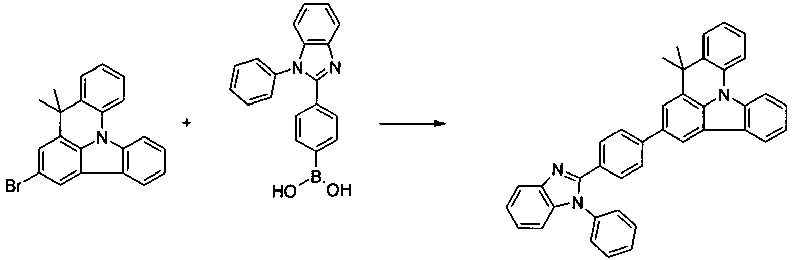

Ein entgaste Suspension von 10.1 g (28 mmol) 6-Brom-8,8-dimethyl-8H-indolo[3,2,1,-de]acridin und 9,42 g (30 mmol) Benzimidazolboronsäure und 7.8 g (31.5 mmol) Kaliumphosphat-hydrat in einem Gemisch aus 7.5 ml Dioxan, 15 ml Toluol und 18 ml Wasser wird unter gutem Rühren mit 0.27 g (0.9 mmol) Tri-o-tolylphosphin und dann mit 33.5 mg (0.15 mmol) Palladium(II)acetat versetzt. Nach 5 h Erhitzen unter Rückfluss lässt man die Mischung erkalten. Der Niederschlag wird abgesaugt, dreimal mit 10 ml Ethanol / Wasser (1:1, v:v) und dreimal mit 5 ml Ethanol gewaschen, anschließend im Vakuum getrocknet und aus Dioxan umkristallisiert. Ausbeute: 12.46 g (22,5 mmol), 81 % d. Th., Reinheit nach ¹H-NMR ca. 99.9 %.

### Beispiel 8: 3-Brom-8,8-diphenyl-8H-indolo[3,2,1-de]acridin

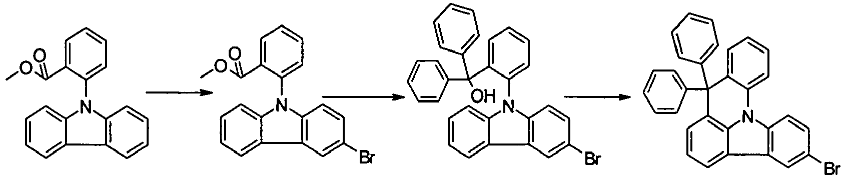

### a) 2-(3-Brom-9H-carbazol)benzoesäuremethylester

62 g (207 mmol) 2-(9H-Carbazol)benzoesäuremethylester werden in 2000 mL DMF auf -10 °C gekühlt, portionsweise mit 37.3 g (207 mmol) NBS versetzt und 6 h bei Raumtemperatur gerührt. Anschließend wird die Mischung mit 500 mL Wasser versetzt und mit CH₂Cl₂ extrahiert. Die organische Phase wird über MgSO₄ getrocknet und das Lösungsmittel im Vakuum entfernt. Das Produkt wird mit Toluol heiß ausgerührt und abgesaugt. Ausbeute: 72 g (190 mmol), 92% d. Th., Reinheit nach ¹H-NMR ca. 98%.

### b) [2-(3-Brom-carbazol-9-yl)-phenyl]-diphenyl-methanol

21.3 g (86.7 mmol) Cer(III)-chlorid werden in 250 ml THF vorgelegt. Zu dieser Lösung wird bei Raumtemperatur 30 g (78.9 mmol) 2-(3-Brom-9*H-*carbazol)benzoesäuremethylester, gelöst in 600 mL getrocknetem THF, zugetropft und 2.5 h gerührt. Die Mischung wird auf 0 °C gekühlt, mit 118.3 ml (236 mmol) 2M Phenylmagnesiumbromid in THF versetzt und über Nacht gerührt. Nach vollständiger Umsetzung wird bei -30 °C vorsichtig mit Methanol gequencht. Die Reaktionslösung wird auf ein Drittel eingeengt, mit 1 L CH₂Cl₂ versetzt, gewaschen und die organische Phase über MgSO₄ getrocknet und eingeengt. Ausbeute: 38.7 g (76.7 mmol), 97 % d. Th., Reinheit nach ¹H-NMR ca. 94%.

### c) 3-Brom-8,8-diphenyl-8H-indolo[3,2,1-de]acridin

38.7 g (76.7 mmol) 2-[2-(3-Bromcarbazol-9-yl)-phenyl]-diphenyl-methanol werden in 750 mL entgastem Dichlormethan gelöst, mit einer Suspension aus 49.6 g Polyphosphorsäure und 33 mL Methansulfonsäure versetzt und für 1 h auf 60 °C erhitzt. Der Ansatz wird abgekühlt und mit Wasser versetzt. Es fällt ein Feststoff aus, der in CH₂Cl₂/THF (1:1) gelöst wird. Die Lösung wird mit 20%iger NaOH vorsichtig alkalisiert, die Phasen werden getrennt und über MgSO₄ getrocknet. Der erhaltene Feststoff wird aus Heptan ausgerührt. Ausbeute: 22 g (45 mmol), 59% d. Th., Reinheit nach ¹H-NMR ca. 95%.

Analog werden folgende Verbindungen erhalten

| **Bsp.** | **Edukt** | **Produkt** | **Ausbeute** |
|---|---|---|---|
| 9 | 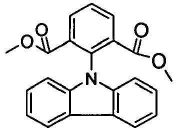 | 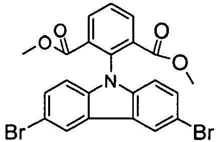 | 63 % |
| 10 | 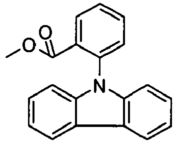 | 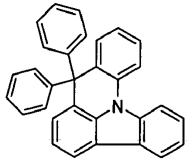 | 71 % |
| 11 | 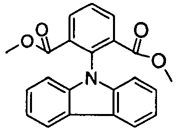 | 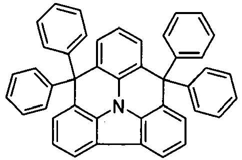 | 59 % |
| 12 | 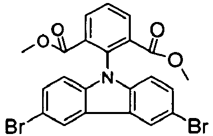 | 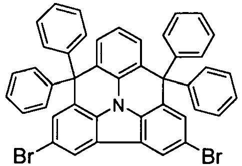 | 56 % |

### Beispiel 13: 8,8-Diphenyl-8H-indolo[3,2,1-de]acridin-6-boronsäure

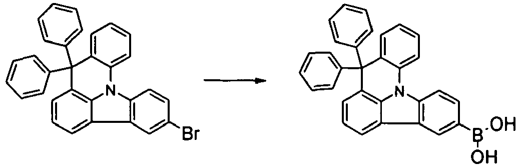

125.9 g (259 mmol) Brom-8,8-diphenyl-8H-indolo[3,2,1-de]acridin werden in 1500 mL trockenem THF gelöst, bei -70 °C 135 mL (337 mmol) einer 2.5 M Lösung von *n*-Butyllithium in Cyclohexan zugetropft, nach 1 h 37 mL Trimethylborat (336 mmol) zugetropft, innerhalb 1 h auf Raumtemperatur erwärmt, das Lösungsmittel entfernt und der Rückstand, der nach ¹H-NMR einheitlich ist, ohne weitere Reinigung in die Folgereaktion eingesetzt. Ausbeute: 87.6 g (194 mmol), 75% d. Th., Reinheit nach ¹H-NMR ca. 96%.

Analog werden folgende Verbindungen erhalten:

| **Bsp.** | **Edukt** | **Produkt** | **Ausbeute** |
|---|---|---|---|
| 14 | 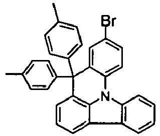 | 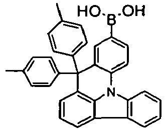 | 61 % |
| | J.Mater.Chem.2009,19,76 61-7665 | | |
| 15 | 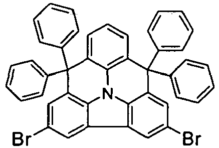 | 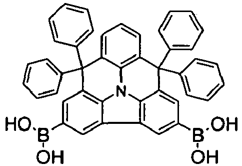 | 55 % |

### Beispiel 16: 6-(4,6-Diphenyl-[1,3,5]triazin-2-yl)-8,8-diphenyl-8H-indolo[3,2,1-de]acridin (nicht Gegenstand der Erfindung)

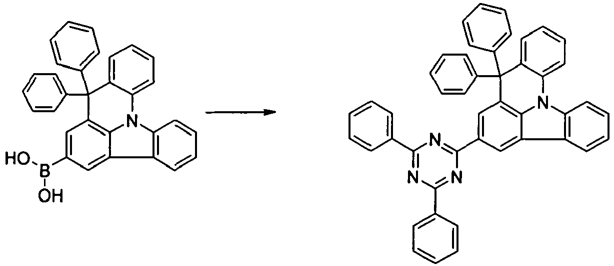

20 g (44 mmol) 8,8-Diphenyl-8H-indolo[3,2,1-de]acridin-6-boronsäure, 11.7 g (44 mmol) 2-Chlor-4,6-diphenyl-1,3,5-triazin und 2.9 g (27.4 mmol) Natriumcarbonat werden in 70 mL Toulol, 70 mL Dioxan und 50 mL Wasser suspendiert. Zu dieser Suspension werden 1.44 mg (1.24 mmol) Pd(PPh₃)₄ gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan / *iso*-Propanol umkristallisiert und abschließend im Hochvakuum sublimiert, Reinheit beträgt 99.9 %. Die Ausbeute beträgt 22.4 g (35 mmol), entsprechend 80 % der Theorie.

Analog werden folgende Verbindungen erhalten:

| **Bsp.** | **Edukt 1** | **Edukt 2** | **Produkt** | **Ausbeute** |
|---|---|---|---|---|
| 17 | 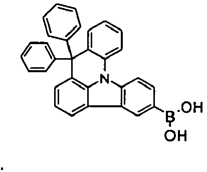 | 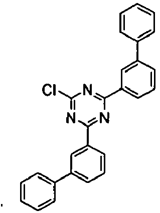 | 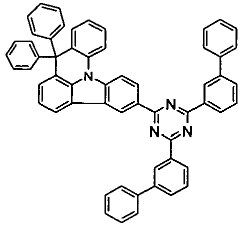 | 67% |
| | | | (nicht Gegenstand der Erfindung) | |
| 18 | 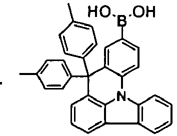 | 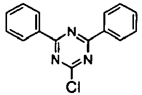 | 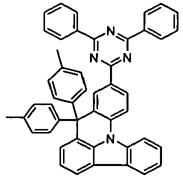 | 72 % |
| | | | (nicht Gegenstand der Erfindung) | |
| 19 | 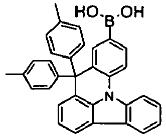 | 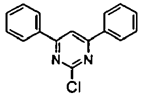 | 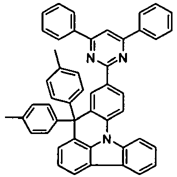 | 61 % |
| | | | (nicht Gegenstand der | |
| | | | Erfindung) | |
| 20 | 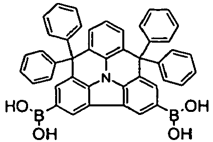 | 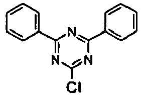 | 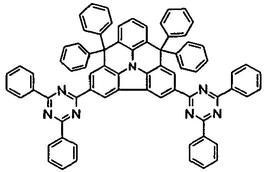 | 66 % |
| | | | (nicht Gegenstand der Erfindung) | |

### Beispiel 21: 8,8-Diphenyl-6-[4-(1-phenyl-1H-benzoimidazol-2-yl)-phenyl]-8H-indolo[3,2,1-de]acridin (nicht Gegenstand der Erfindung)

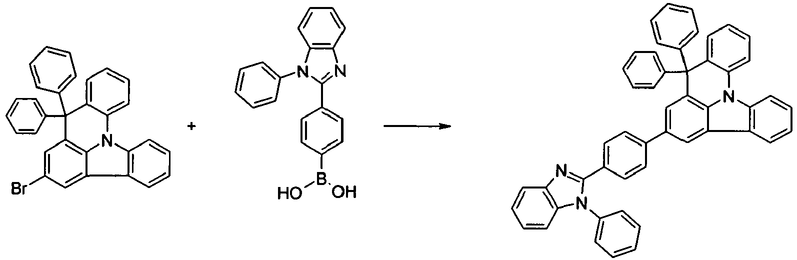

Ein entgaste Suspension von 13.6 g (28 mmol) 3-Brom-8,8-diphenyl-8H-indolo[3,2,1-de]acridin, 9.42 g (30 mmol) Benzimidazolboronsäure und 7.8 g (31.5 mmol) Kaliumphosphat-hydrat in einem Gemisch aus 7.5 ml Dioxan, 15 ml Toluol und 18 ml Wasser wird unter gutem Rühren mit 0.27 g (0.9 mmol) Tri-o-tolylphosphin und dann mit 33.5 mg (0.15 mmol) Palladium(II)acetat versetzt. Nach 5 h Erhitzen unter Rückfluss lässt man die Mischung erkalten. Der Niederschlag wird abgesaugt, dreimal mit 10 ml Ethanol / Wasser (1:1, v:v) und dreimal mit 5 ml Ethanol gewaschen, anschließend im Vakuum getrocknet und aus Dioxan umkristallisiert. Ausbeute: 16 g (23 mmol), 85 % d. Th., Reinheit nach ¹H-NMR ca. 99.9 %.

Analog werden folgende Verbindungen erhalten

| **Bsp.** | **Edukt** | **Produkt** | **Ausbeute** |
|---|---|---|---|
| 22 | 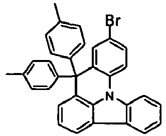 | 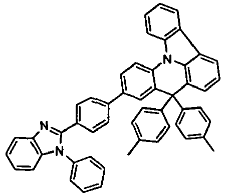 | 73 % |
| | J.Mater.Chem.2009,19,76 61-7665 | (nicht Gegenstand der Erfindung) | |
| 23 | 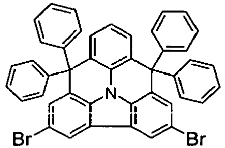 | 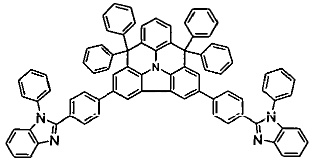 | 65 % |
| | | (nicht Gegenstand der Erfindung) | |

### Beispiel 24: (8,8-Diphenyl-8H-indolo[3,2,1-de]acridin-3-yl)-phenyl-methanon (nicht Gegenstand der Erfindung)

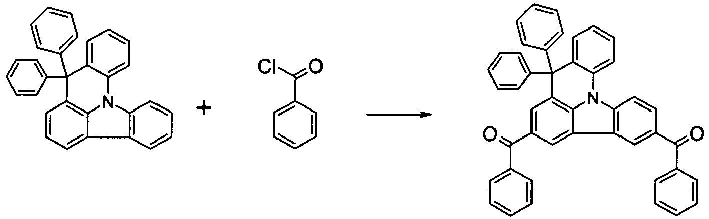

Eine entgaste Lösung von 6.1 g (18 mmol) 8,8-Diphenyl-8H-indolo[3,2,1-de]acridin in 40 ml Chloroform wird auf 0 °C gekühlt und mit 5 g (37 mmol) AlCl₃ versetzt. Dann wird bei dieser Temperatur 3.9 g Benzoylchlorid zugetropft und 8 h gerührt. Die Mischung wird mit 50 ml Wasser versetzt, die organische Phase wird abgetrennt, über Kieselgel filtriert und zur Trockene eingeengt. Der Rückstand wird aus Toluol und aus Dichlormethan / *iso-*Propanol umkristallisiert und abschließend im Hochvakuum sublimiert, Reinheit beträgt 99.9 %. Die Ausbeute beträgt 13.7 g (27 mmol), entsprechend 90 % der Theorie.

Analog werden folgende Verbindungen erhalten

| **Bsp.** | **Edukt 1** | **Edukt 2** | **Produkt** | **Ausbeute** |
|---|---|---|---|---|
| 25 | 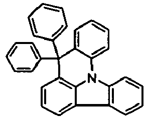 | 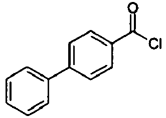 | 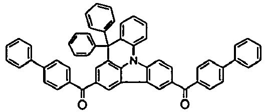 | 72 % |
| | | | (nicht Gegenstand der Erfindung) | |
| 26 | 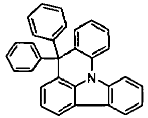 | 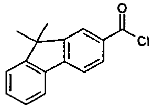 | 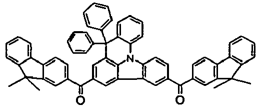 | 83 % |
| | | | (nicht Gegenstand der Erfindung) | |

### Beispiel 27: Bis-(8,8-dimethyl-8H-indolo[3,2,1-d,e]acridin-3-yl)-methanon

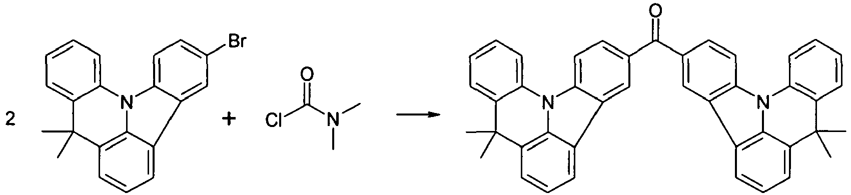

Eine Lösung von 3-Brom-8,8-dimethyl-8H-indolo[3,2,1-de]acridin (15.4 g, 43 mmol) in THF (250 mL) wird bei -78 °C mit *n*-Butyllithium (26.6 mL einer 2.0 N Lösung in Hexan) versetzt, 1 h bei dieser Temperatur gerührt und mit einer Lösung von Dimethylcarbamoylchlorid (2.0 mL, 21 mmol) in THF (2 mL) versetzt. Das Reaktionsgemisch wird nach weiteren 2 h Rühren bei -78 °C langsam auf Raumtemperatur erwärmt und auf Eiswasser gegeben. Der entstandene Niederschlag wird durch Filtration abgetrennt und durch mehrfache Umkristallisation mit Dioxan aufgereinigt. Abschließende Sublimation im Hochvakuum (T = 350 °C, p = 7 x 10⁻⁵ mbar) ergibt das Produkt in einer Reinheit von 99.9 % (5.2 g, 20 %).

### Beispiel 28: Herstellung der OLEDs

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, verwendete Materialien) angepasst wird.

In den folgenden Beispielen 29 bis 55 (siehe Tabellen 1 bis 3) werden die Ergebnisse verschiedener OLEDs vorgestellt. Glasplättchen, die mit strukturiertem ITO (Indium Zinn Oxid) der Dicke 150 nm beschichtet sind werden zur verbesserten Prozessierung mit 20 nm PEDOT beschichtet (Poly(3,4-ethylendioxy-2,5-thiophen), aus Wasser aufgeschleudert; bezogen von H. C. Starck, Goslar, Deutschland). Diese beschichteten Glasplättchen bilden die Substrate, auf welche die OLEDs aufgebracht werden. Die OLEDs haben prinzipiell folgenden Schichtaufbau: Substrat / Lochtransportschicht (HTL) / Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs verwendeten Materialien sind in Tabelle 2 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H3:CBP:TER1 (55%:35%:10%) bedeutet hierbei, dass das Material H3 in einem Volumenanteil von 55 %, CBP in einem Anteil von 35 % und TER1 in einem Anteil von 10 % in der Schicht vorliegt. Analog kann auch die Elektronentransportschicht aus einer Mischung zweier Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Helligkeits-Kennlinien (IUL-Kennlinien) sowie die Lebensdauer bestimmt. Als Lebensdauer wird die Zeit definiert, nach der die Leuchtdichte von einer bestimmten Startleuchtdichte aus auf einen gewissen Anteil abgesunken ist. Die Angabe LD80 bedeutet, dass es sich bei der genannten Lebensdauer um die Zeit handelt, bei der die Leuchtdichte auf 80 % der Startleuchtdichte abgefallen ist, also von z. B. 4000 cd/m² auf 3200 cd/m².

Im Folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 3 gezeigten Daten darstellt. Wie sich der Tabelle entnehmen lässt, werden auch bei Verwendung der nicht näher ausgeführten erfindungsgemäßen Verbindungen Verbesserungen gegenüber dem Stand der Technik erzielt, teilweise in allen Parametern, in manchen Fällen ist nur eine Verbesserung von Effizienz oder Spannung oder Lebensdauer zu beobachten. Allerdings stellt bereits die Verbesserung eines der genannten Paramter einen signifikanten Fortschritt dar, weil verschiedene Anwendungen die Optimierung hinsichtlich unterschiedlicher Parameter erfordern.

### Verwendung als Matrixmaterialien in phosphoreszierenden OLEDs

Die Verbindungen werden erfindungsgemäß als Matrixmaterialien (Hostmaterialien, Wirtsmaterialen) für phosphoreszierende Dotanden einsetzen. Hierbei kommen die Verbindungen H2 und H3 zum Einsatz. Als Vergleich gemäß dem Stand der Technik werden die Verbindung H1 und H4 verwendet. Es werden OLEDs mit dem grün emittierenden Dotanden TEG1 sowie dem rot emittierenden Dotanden TER1 gezeigt. Die Ergebnisse der OLEDs sind in Tabelle 3 zusammengefasst. Die Bsp. 29 bis 32A zeigen OLEDs mit Materialien gemäß dem Stand der Technik und dienen als Vergleichsbeispiele.

Die Vorteile beim Einsatz von erfindungsgemäßen Verbindungen als Matrixmaterialien für rot und grün emittierende OLEDs sind eine Steigerung der Lebensdauer bei gleichzeitiger Verringerung der Betriebsspannung und einer damit verbundenen signifikanten Erhöhung der Leistungseffizienz (siehe Bsp. 33-38). So erhält man bei Einsatz von H3 eine um 55 % erhöhte Lebensdauer gegenüber dem Stand der Technik H1, wobei sich die Leistungseffizienz ebenfalls sehr deutlich, nämlich um etwa 40 %, verbessert (vgl. Bsp. 38 und 32). Bei Einsatz von H2 in grün emittierenden OLEDs ist die Verbesserung in diesen Parametern ebenfalls sehr deutlich, fällt aber etwas geringer aus als bei H3. Gegenüber dem Stand der Technik H4 zeigen die erfindungsgemäßen Verbindungen noch deutlich höhere Verbesserungen in Bezug auf Effizienz, Spannung und Lebensdauer.

Im Falle roter Emission zeigt H2 etwas bessere Kenndaten als H3, die Leistungseffizienz erhöht sich hierbei um bis zu 30 %, wobei die Lebensdauer gegenüber dem Stand der Technik um etwa 35 % verbessert ist (vgl. Bsp. 35 und 30).

Insbesondere zeigen auch Verbindungen, die mit Phenylringen an der Brücke Y substituiert sind, gute Leistungsdaten. So zeigt z. B. H5 im Vergleich zu H3 eine bessere Lebensdauer bei fast identischer Leistungseffizienz (Beispiele 41 und 47). Das gleiche gilt für den Vergleich von H2 mit H9 (Beispiele 35 und 36).

**Tabelle 1: Aufbau der OLEDs**

| Bsp. | HTL Dicke | EBL Dicke | EML Dicke | HBL Dicke | ETL Dicke | EIL Dicke |
|---|---|---|---|---|---|---|
| 29 (Vgl.) | HTM1 20 nm | NPB 20 nm | H1:TER1 (85%:15%) 30 nm | --- | Alq₃ 20 nm | LiF 1 nm |
| 30 (Vgl.) | HTM1 20 nm | NPB 20 nm | H1:CBP:TER1 (45%:45%:10%) 30 nm | H1 10 nm | Alq₃ 20 nm | LiF 1 nm |
| 31 (Vgl.) | HTM1 160 nm | EBM1 20 nm | H1:TEG1 (90%:10%) 30 nm | H1 10 nm | ETM1:LiQ (50%:50%) 40 nm | --- |
| 32 (Vgl.) | HTM1 160 nm | EBM1 20 nm | H1:TEG1 (90%:10%) 30 nm | --- | ETM1:LiQ (50%:50%) 40 nm | --- |
| 32A (Vgl.) | HTM1 160 nm | EBM1 20 nm | H4:TEG1 (90%:10%) 30 nm | --- | ETM1:LiQ (50%:50%) 40 nm | --- |
| 33* | HTM1 20 nm | NPB 20 nm | H2:TER1 (85%:15%) 30 nm | --- | Alq₃ 20 nm | LiF 1 nm |
| 34* | HTM1 20 nm | NPB 20 nm | H3:TER1 (85%:15%) 30 nm | --- | Alq₃ 20 nm | LiF 1 nm |
| 35* | HTM1 20 nm | NPB 20 nm | H2:CBP:TER1 (45%:45%:10%) 30 nm | H1 10 nm | Alq₃ 20 nm | LiF 1 nm |
| 36* | HTM1 160 nm | EBM1 20 nm | H2:TEG1 (90%:10%) 30 nm | H1 10 nm | ETM1:LiQ (50%:50%) 40 nm | --- |
| 37* | HTM1 160 nm | EBM1 20 nm | H3:TEG1 (90%:10%) 30 nm | H1 10 nm | ETM1:LiQ (50%:50%) 40 nm | --- |
| 38* | HTM1 160 nm | EBM1 20 nm | H3:TEG1 (90%:10%) 30 nm | --- | ETM1:LiQ (50%:50%) 40 nm | --- |
| 39* | HTM1 20 nm | NPB 20 nm | H1:TER1 (85%:15%) 30 nm | --- | ETM2 20 nm | LiF 1 nm |
| 40* | HTM1 160 nm | EBM1 20 nm | H1:TEG1 (90%:10%) 30 nm | --- | ETM2:LiQ (50%:50%) 40 nm | --- |
| 41* | HTM1 160 nm | EBM1 20 nm | H5:TEG1 (90%:10%) 30 nm | H1 10 nm | ETM1:LiQ (50%:50%) 40 nm | --- |
| 42* | HTM1 160 nm | EBM1 20 nm | H6:TEG1 (90%:10%) 30 nm | H1 10 nm | ETM1:LiQ (50%:50%) 40 nm | --- |
| 43* | HTM1 20 nm | NPB 20 nm | H7:TER1 (85%:15%) 30 nm | --- | Alq₃ 20 nm | LiF 1 nm |
| 44* | HTM1 20 nm | NPB 20 nm | H8:TER1 (85%:15%) 30 nm | --- | Alq₃ 20 nm | LiF 1 nm |
| 45* | HTM1 160 nm | EBM1 20 nm | H9:TEG1 (90%:10%) 30 nm | H1 10 nm | ETM1:LiQ (50%:50%) 40 nm | --- |
| 46* | HTM1 160 nm | EBM1 20 nm | H10:TEG1 (90%:10%) 30 nm | H1 10 nm | ETM1:LiQ (50%:50%) 40 nm | --- |
| 47* | HTM1 160 nm | EBM1 20 nm | H11:TEG1 (90%:10%) 30 nm | H1 10 nm | ETM1:LiQ (50%:50%) 40 nm | --- |
| 48* | HTM1 160 nm | EBM1 20 nm | H12:TEG1 (90%:10%) 30 nm | H1 10 nm | ETM1:LiQ (50%:50%) 40 nm | --- |
| 49* | HTM1 160 nm | EBM1 20 nm | H13:TEG1 (90%:10%) 30 nm | H1 10 nm | ETM1:LiQ (50%:50%) 40 nm | --- |
| 50* | HTM1 160 nm | EBM1 20 nm | H14:TEG1 (90%:10%) 30 nm | H1 10 nm | ETM1:LiQ (50%:50%) 40 nm | --- |
| 51* | HTM1 20 nm | NPB 20 nm | H15:TER1 (85%:15%) 30 nm | --- | Alq₃ 20 nm | LiF 1 nm |
| 52 | HTM1 160 nm | EBM1 20 nm | H16:TEG1 (90%:10%) 30 nm | H1 10 nm | ETM1:LiQ (50%:50%) 40 nm | --- |
| 53* | HTM1 160 nm | EBM1 20 nm | H14:IC1:TEG1 (30%:60%:10%) 30 nm | H1 10 nm | ETM1:LiQ (50%:50%) 40 nm | --- |
| 54 | HTM1 160 nm | EBM1 20 nm | H16:IC1:TEG1 (30%:60%:10%) 30 nm | H1 10 nm | ETMI:LiQ (50%:50%) 40 nm | --- |
| 55* | HTM1 160 nm | EBM1 20 nm | H1:TEG1 (90%:10%) 30 nm | --- | H11:LiQ (50%:50%) 40 nm | --- |

| | | | | | | |
|---|---|---|---|---|---|---|
| * Referenz (nicht erfindungsgemäß) | | | | | | |

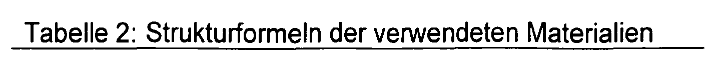

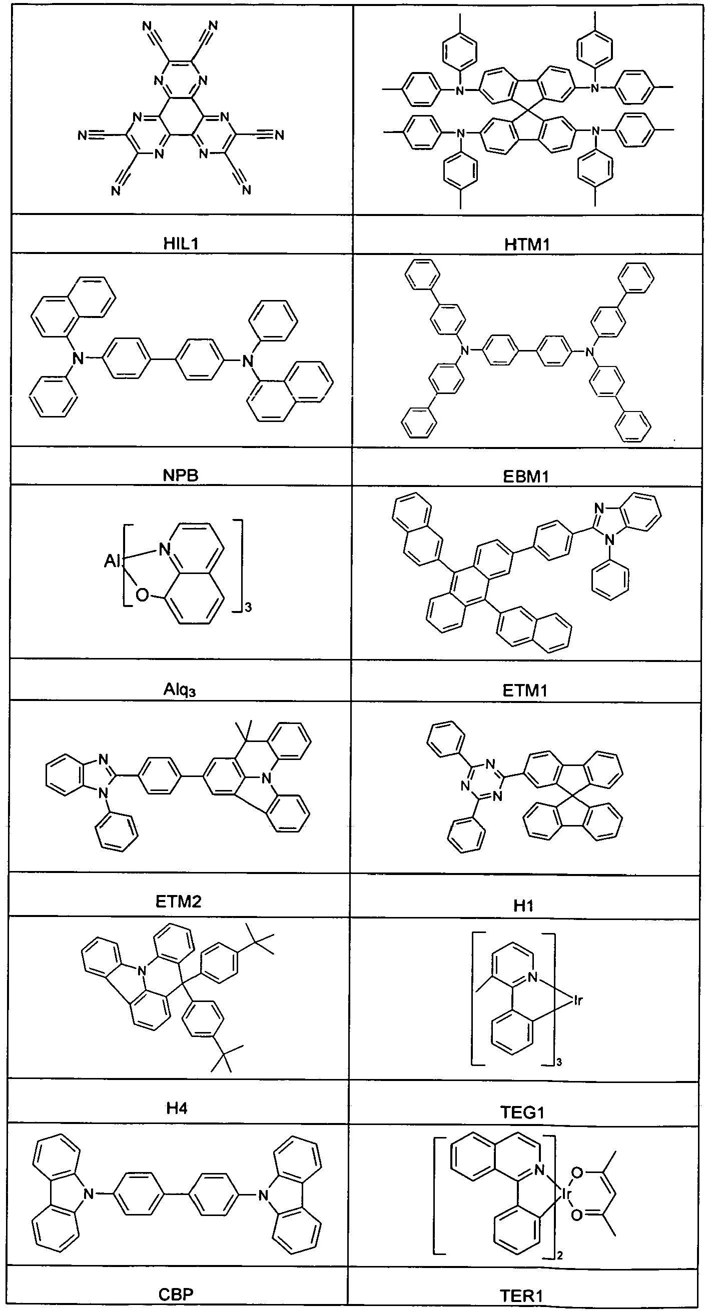

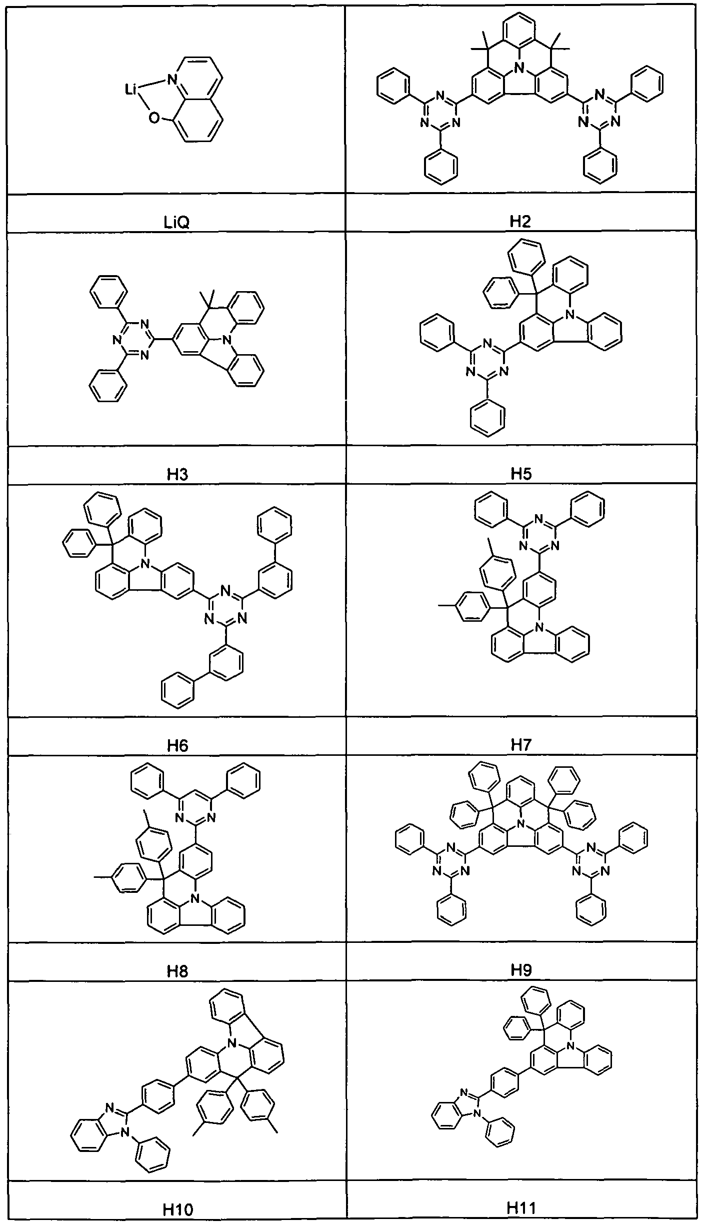

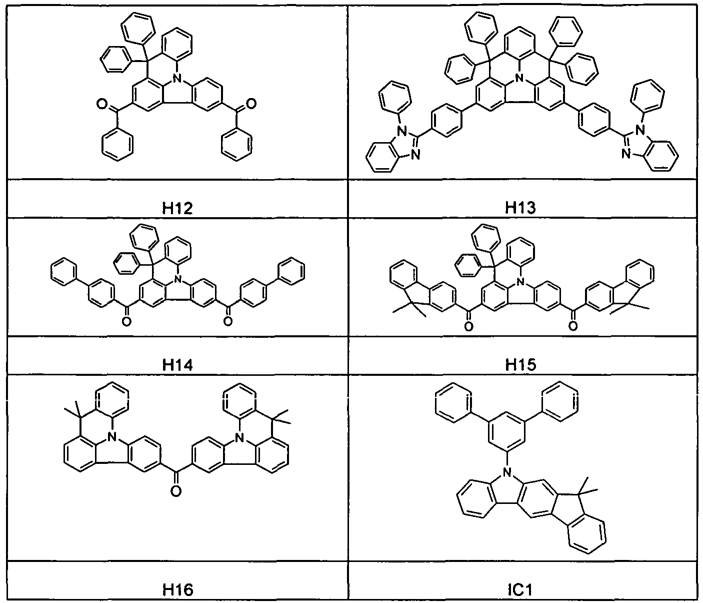

**Tabelle 3: Verwendung von erfindungsgemäßen Verbindungen als Matrixmaterialien und ETM in phosphoreszenten OLEDs**

| Bsp. | Spannung für 1000 cd/m2 | Effizienz bei 1000 cd/m2 | Effizienz bei 1000 cd/m² | CIE x/y bei 1000 cd/m² | LD80 ab 4000 cd/m² |
|---|---|---|---|---|---|
| 29 (Vgl.) | 5.0 V | 7.2 cd/A | 4.5 lm/W | 0.69/0.31 | 230 h |
| 30 (Vgl.) | 5.2 V | 8.1 cd/A | 4.9 lm/W | 0.68/0.32 | 250 h |
| 31 (Vgl.) | 4.7 V | 55 cd/A | 37 lm/w | 0.36/0.61 | 440 h |
| 32 (Vgl.) | 4.6 V | 54 cd/A | 37 lm/W | 0.37/0.60 | 400 h |
| 32A (Vgl.) | 4.9 V | 46 cd/A | 30 lm/W | 0.37/0.60 | 270 h |
| 33* | 4.6 V | 8.4 cd/A | 5.7 Im/W | 0.69/0.31 | 310 h |
| 34* | 4.6 V | 8.2 cd/A | 5.6 lm/W | 0.68/0.31 | 330 h |
| 35* | 4.8 V | 9.8 cd/A | 6.4 lm/W | 0.69/0.32 | 340 h |
| 36* | 3.7 V | 59 cd/A | 50 lm/W | 0.36/0.60 | 590 h |
| 37* | 3.6 V | 58 cd/A | 51 lm/W | 0.36/0.61 | 670 h |
| 38* | 3.4 V | 55 cd/A | 51 lm/W | 0.36/0.61 | 620 h |
| 39* | 4.3 V | 7.9 cd/A | 5.8 lm/W | 0.69/0.32 | 250 h |
| 40* | 4.3 V | 61 cd/A | 45 lm/W | 0.37/0.61 | 410 h |
| 41* | 3.5 V | 56 cd/A | 50 lm/W | 0.36/0.61 | 740 h |
| 42* | 3.8 V | 52 cd/A | 44 lm/W | 0.36/0.60 | 710 h |
| 43* | 4.7 V | 8.5 cd/A | 5.7 lm/W | 0.69/0.31 | 290 h |
| 44* | 4.4 V | 7.8 cd/A | 5.5 lm/W | 0.69/0.31 | 360 h |
| 45* | 3.7 V | 58 cd/A | 49 lm/W | 0.36/0.61 | 660 h |
| 46* | 4.4 V | 52 cd/A | 37 lm/W | 0.36/0.61 | 510 h |
| 47* | 4.5 V | 49 cd/A | 34 lm/W | 0.36/0.60 | 480 h |
| 48* | 4.0 V | 57 cd/A | 44 lm/W | 0.36/0.60 | 530 h |
| 49* | 4.6 V | 47 cd/A | 32 lm/W | 0.3610.60 | 420 h |
| 50* | 3.9 V | 55 cd/A | 44 lm/W | 0.36/0.61 | 620 h |
| 51* | 4.8 V | 7.9 cd/A | 5.2 lm/W | 0.69/0.31 | 380 h |
| 52 | 3.4 V | 57 cd/A | 53 lm/W | 0.3610.60 | 640 h |
| 53* | 4.1 V | 53 cd/A | 41 lm/W | 0.36/0.61 | 700 h |
| 54 | 3.5 V | 53 cd/A | 47 lm/W | 0.36/0.61 | 730 h |
| 55* | 4.3 V | 63 cd/A | 45 lm/W | 0.36/0.61 | 450 h |

| | | | | | |
|---|---|---|---|---|---|
| * Referenz (nicht erfindungsgemäß) | | | | | |

## Patentansprüche

1. Organische Elektrolumineszenzvorrichtung enthaltend eine Verbindung gemäß der Formel (17) oder Formel (18) als Matrixmaterial für phosphoreszierende Emitter, 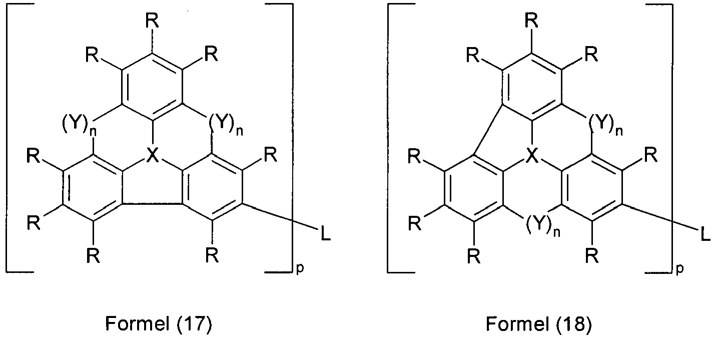 wobei für die verwendeten Symbole und Indizes gilt:
X ist bei jedem Auftreten N;
Y ist gleich oder verschieden bei jedem Auftreten C(R)₂;
R ist bei jedem Auftreten gleich oder verschieden ausgewählt aus der Gruppe bestehend aus H, D, F, Cl, Br, I, CN, NO₂, N(Ar)₂, N(R¹)₂, C(=O)_{Ar}, C(=O)R¹, P(=O)(Ar)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R¹ substituiert sein kann, wobei eine oder mehrere nicht-benachbarte CH₂-Gruppen durch R¹C=CR¹ C≡C, Si(R¹)₂, Ge(R¹)₂, Sn(R¹)₂, C=O, C=S, C=Se, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 80 aromatischen Ringatomen, das jeweils mit einem oder mehreren Resten R¹ substituiert sein kann, einer Aryloxy- oder Heteroaryloxygruppe mit 5 bis 60 aromatischen Ringatomen, die mit einem oder mehreren Resten R¹ substituiert sein kann, oder einer Kombination dieser Systeme, wobei optional zwei oder mehr benachbarte Substituenten R ein monocyclisches oder polycyclisches, aliphatisches, aromatisches oder heteroaromatisches Ringsystem bilden können, das mit einem oder mehreren Resten R¹ substituiert sein kann; mit der Maßgabe, dass die Gruppen R, die in Y gebunden sind, gleich oder verschieden bei jedem Auftreten ausgewählt sind aus der Gruppe bestehend aus Alkylgruppen mit 1 bis 10 C-Atomen oder aromatischen oder heteroaromatischen Ringsystemen mit 5 bis 20 aromatischen Ringatomen, welche jeweils durch einen oder mehrere Reste R¹ substituiert sein können;
R¹ ist bei jedem Auftreten gleich oder verschieden ausgewählt aus der Gruppe bestehend aus H, D, F, Cl, Br, I, CN, NO₂, N(Ar)₂, N(R²)₂, C(=O)_{Ar}, C(=O)R², P(=O)(Ar)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C=C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R² substituiert sein kann, einer Aryloxy- oder Heteroaryloxygruppe mit 5 bis 60 aromatischen Ringatomen, die mit einem oder mehreren Resten R² substituiert sein kann, oder einer Kombination dieser Systeme, wobei optional zwei oder mehr benachbarte Substituenten R ein monocyclisches oder polycyclisches, aliphatisches, aromatisches oder heteroaromatisches Ringsystem bilden können, das mit einem oder mehreren Resten R² substituiert sein kann;
R² ist ausgewählt aus der Gruppe bestehend aus H, D, F, CN, einem aliphatischem Kohlenwasserstoffrest mit 1 bis 20 C-Atomen, einem aromatischem oder heteroaromatischem Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R² miteinander ein mono- oder polycyclisches, aliphatisches, aromatisches oder heteroaromatisches Ringsystem bilden können;
Ar ist bei jedem Auftreten gleich oder verschieden ein aromatisches oder heteroaromatisches Ringsystem mit 5-30 aromatischen Ringatomen, das mit einem oder mehreren nicht-aromatischen Resten R² substituiert sein kann; dabei können zwei Reste Ar, welche an dasselbe N-Atom oder P-Atom binden, auch durch eine Einfachbindung oder eine Brücke, ausgewählt aus N(R²), C(R²)₂ oder O, miteinander verbrückt sein;
L ist eine bivalente oder höher valente geradkettige Alkylen-, Alkyliden-, Alkylenoxy- oder Thioalkylenoxygruppe mit 1 bis 40 C-Atomen oder eine verzweigte oder cyclische Alkylen-, Alkyliden-, Alkylenoxy- oder Thioalkylenoxygruppe mit 3 bis 40 C-Atomen oder eine Alkenylen- oder Alkinylengruppe mit 2 bis 40 C-Atomen, die mit jeweils einem oder mehreren Resten R¹ substituiert sein kann, wobei eine oder mehrere nicht benachbarte CH₂-Gruppen durch -R¹C=CR¹-, -C≡C-, Si(R¹)₂, Ge(R¹)₂, Sn(R¹)₂, C=O, C=S, C=Se, C=NR¹, P(=O)R¹, S=O, SO₂, -O-, -S- oder -CONR¹- ersetzt sein können und wobei ein oder mehrere H-Atome durch D, F, Cl, Br, I, CN oder NO₂ ersetzt sein können, oder ein mindestens bivalentes aromatisches oder heteroaromatisches Ringsystem mit 5 bis 80 aromatischen Ringatomen, welches durch einen oder mehrere Reste R¹ substituiert sein kann, oder P(R¹)₃₋ₚ, P(=O)(R¹)₃₋ₚ, C(R¹)₄₋ₚ, Si(R¹)₄₋ₚ, N(Ar)₃₋ₚ oder eine Kombination aus zwei, drei, vier oder fünf dieser Systeme; oder L ist eine chemische Bindung;
n ist bei jedem Auftreten gleich oder verschieden 0 oder 1, wobei für n = 0 statt Y ein Wasserstoff oder Rest R¹ vorhanden ist, mit der Maßgabe, dass pro Einheit mindestens ein Index n = 1 ist;
p ist 2, 3, 4, 5 oder 6, mit der Maßgabe, dass p nicht größer ist als die maximale Valenz von L;
**dadurch gekennzeichnet, dass** mindestens ein Rest R für eine der Gruppen der folgenden Formel (3) steht, 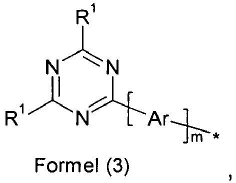 und/oder dass mindestens eine Gruppe L für eine Gruppe der folgenden Formeln (10) oder (11) steht, 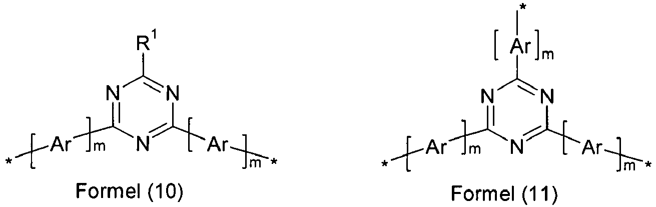 wobei R¹ für ein aromatisches oder heteroaromatisches Ringsystem mit 5 bis 20 aromatischen Ringatomen steht, das keine kondensierten Aromaten mit mehr als 10 aromatischen Ringatomen aufweist und welches jeweils durch einen oder mehrere Reste R² substituiert sein kann, und die weiteren verwendeten Symbole die oben genannten Bedeutungen haben und der Index m für 0 oder 1 steht; * deutet dabei die Position der Bindung der Gruppe gemäß Formel (3), (10) bzw. (11) an.

2. Organische Elektrolumineszenzvorrichtung nach Anspruch 1 wobei die Verbindung der Formel (17) oder (18) ausgewählt ist aus den Verbindungen gemäß den Formeln (20), (21), (23) oder (24), 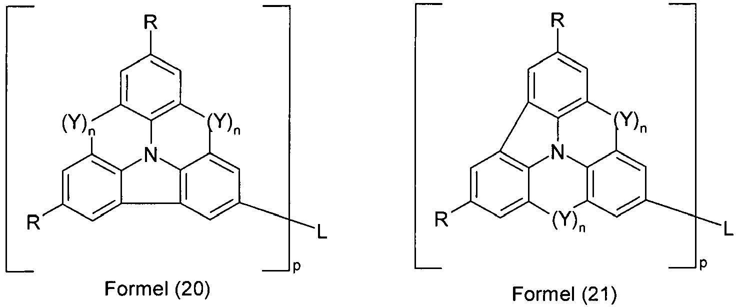 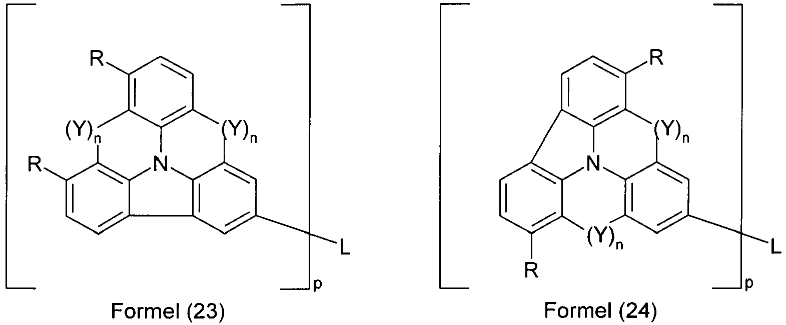 wobei Y gleich oder verschieden bei jedem Auftreten für C(R)₂, die als nicht substituiert gezeichneten C-Atome auch durch D statt H substituiert sein können und die weiteren Symbole und Indizes die in Anspruch 1 genannten Bedeutungen aufweisen.

3. Organische Elektrolumineszenzvorrichtung nach Anspruch 1 oder 2, **dadurch gekennzeichnet, dass** L eine mindestens bivalente geradkettige Alkylengruppe mit 1 bis 10 C-Atomen oder eine verzweigte oder cyclische Alkylengruppe mit 3 bis 10 C-Atomen, die mit jeweils einem oder mehreren Resten R¹ substituiert sein kann, wobei ein oder mehrere H-Atome durch D oder F ersetzt sein können, oder ein mindestens bivalentes aromatisches oder heteroaromatisches Ringsystem mit 5 bis 24 aromatischen Ringatomen ist, welches durch einen oder mehrere Reste R¹ substituiert sein kann; oder L ist eine chemische Bindung; oder L ist eine Gruppe gemäß einer der Formeln (10) oder (11).

4. Organische Elektrolumineszenzvorrichtung nach einem oder mehreren der Ansprüche 1 bis 3, **dadurch gekennzeichnet, dass** R gleich oder verschieden bei jedem Auftreten ausgewählt ist aus der Gruppe bestehend aus H, D, F, Cl, Br, CN, N(Ar)₂, C(=O)_{Ar}, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenyl- oder Alkinylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R¹ substituiert sein kann, einer Aryloxy- oder Heteroaryloxygruppe mit 5 bis 30 aromatischen Ringatomen, die mit einem oder mehreren Resten R¹ substituiert sein kann, oder einer Kombination dieser Systeme, wobei wenigstens ein Rest R eine Gruppe der Formel (3) darstellt; mit der Maßgabe, dass die Gruppen R, die in Y gebunden sind, gleich oder verschieden bei jedem Auftreten ausgewählt sind aus der Gruppe bestehend aus Alkylgruppen mit 1 bis 10 C-Atomen oder aromatischen oder heteroaromatischen Ringsystemen mit 5 bis 20 aromatischen Ringatomen, welche jeweils durch einen oder mehrere Reste R¹ substituiert sein können.

5. Organische Elektrolumineszenzvorrichtung nach einem oder mehreren der Ansprüche 1 bis 4, wobei die Verbindung der Formel (17) oder (18) ausgewählt ist aus den Verbindungen gemäß den Formeln (41), (42), (44), (45), (47) bis (50), (52), (53), (55), (57) und (58), 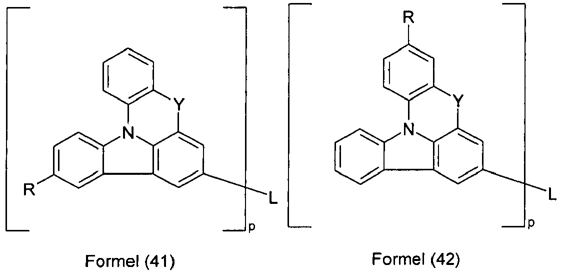 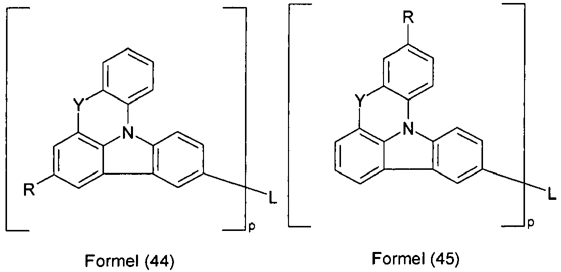 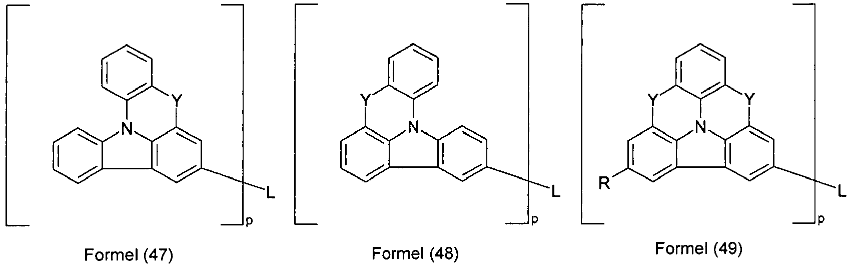 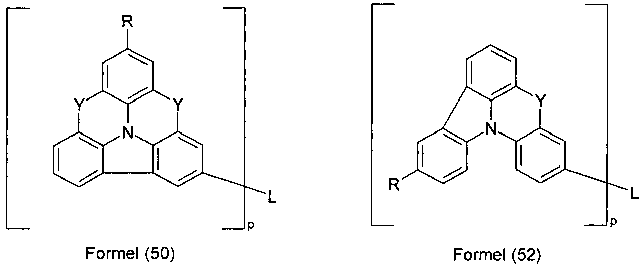 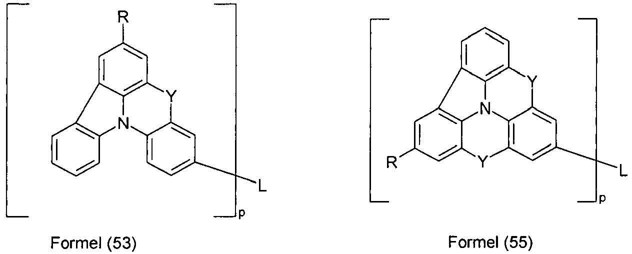 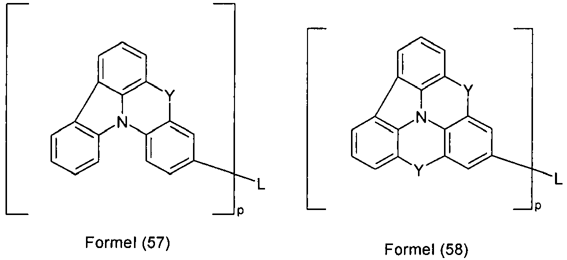 wobei R eine Gruppe gemäß Formel (3) darstellt und wobei L in Formel (47), (48), (57) oder (58) eine Gruppe gemäß einer der Formeln (10) oder (11) darstellt, Y gleich oder verschieden bei jedem Auftreten für C(R)₂ steht, wobei R, welches in der C(R)₂-Gruppe gebunden ist, gleich oder verschieden bei jedem Auftreten für eine Alkylgruppe mit 1 bis 10 C-Atomen oder ein aromatisches oder heteroaromatisches Ringsystem mit 5 bis 18 aromatischen Ringatomen und bevorzugt mindestens einer der Reste R in diesen Gruppen ein aromatisches oder heteroaromatisches Ringsystem mit 5 bis 18 aromatischen Ringatomen darstellt, welches jeweils durch einen oder mehrere Reste R¹ substituiert sein kann, steht; weiterhin können die als nicht substituiert gezeichneten C-Atome auch durch D statt H substituiert sein, und die weiteren Symbole und Indizes weisen die in Anspruch 1 genannten Bedeutungen auf.

6. Organische Elektrolumineszenzvorrichtung nach einem oder mehreren der Ansprüche 1 bis 4, **dadurch gekennzeichnet, dass** die Strukturen der Formeln (17), (18), (20) und (21) jeweils in der Position para zum zentralen Atom X einen Rest R aufweisen, wobei die Substituenten R in der para-Position von X, die nicht für eine Gruppe der Formel (3) stehen, für eine Alkylgruppe mit 1 bis 10 C-Atomen, insbesondere mit 1 bis 4 C-Atomen, oder für ein aromatisches oder heteroaromatisches Ringsystem mit 5 bis 24 aromatischen Ringatomen, welches durch einen oder mehrere Reste R¹ substiutiert sein kann, insbesondere für eine Phenylgruppe, welche durch einen oder mehrere Reste R¹ substituiert sein kann, stehen.

7. Organische Elektrolumineszenzvorrichtung nach einem oder mehreren der Ansprüche 1 bis 6, **dadurch gekennzeichnet, dass** in den Strukturen der Formeln (3), (10) und (11) das Symbol R¹ für Phenyl, ortho-, meta- oder para-Biphenyl, ortho-, meta- oder para-Terphenyl, Quaterphenyl oder 1- oder 2-Naphthyl steht, welches jeweils durch einen oder mehrere Reste R² substituiert sein kann.

8. Organische Elektrolumineszenzvorrichtung nach einem oder mehreren der Ansprüche 1 bis 7, **dadurch gekennzeichnet, dass** die Gruppe Ar in den Formeln (3), (10) und (11) für ein aromatisches oder heteroaromatisches Ringsystem mit 5 bis 20 aromatischen Ringatomen, das keine kondensierten Aromaten mit mehr als 10 aromatischen Ringatomen aufweist und welches mit einem oder mehreren nicht-aromatischen Resten R¹ substituiert sein kann, steht.

9. Verwendung einer Verbindung gemäß der Formel (17) oder Formel (18), wie in einem oder mehreren der Ansprüche 1 bis 8 definiert, als Matrixmaterial für phosphoreszierende Emitter in einer organischen Elektrolumineszenzvorrichtung.

## Claims

1. Organic electroluminescent device containing a compound of the formula (17) or formula (18) as matrix material for phosphorescent emitters, 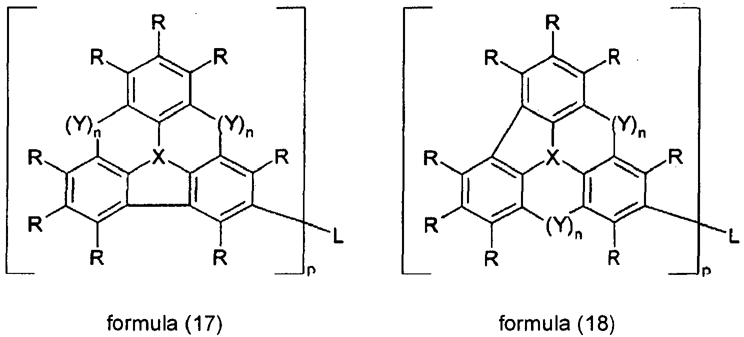 where the following applies to the symbols and indices used:
X is on each occurrence N;
Y is, identically or differently on each occurrence, C(R)₂;
R is selected on each occurrence, identically or differently, from the group consisting of H, D, F, Cl, Br, I, CN, NO₂, N(Ar)₂, N(R¹)₂, C(=O)Ar, C(=O)R¹, P(=O)(Ar)₂, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may in each case be substituted by one or more radicals R¹, where one or more non-adjacent CH₂ groups may be replaced by R¹C=CR¹, C≡C, Si(R¹)₂, Ge(R¹)₂, Sn(R¹)₂, C=O, C=S, C=Se, C=NR¹, P(=O)(R¹), SO, SO₂, NR¹, O, S or CONR¹ and where one or more H atoms may be replaced by D, F, Cl, Br, I, CN or NO₂, an aromatic or heteroaromatic ring system having 5 to 80 aromatic ring atoms, which may in each case be substituted by one or more radicals R¹, an aryloxy or heteroaryloxy group having 5 to 60 aromatic ring atoms, which may be substituted by one or more radicals R¹, or a combination of these systems, where two or more adjacent substituents R may optionally form a monocyclic or polycyclic, aliphatic, aromatic or heteroaromatic ring system, which may be substituted by one or more radicals R¹; with the proviso that groups R which are bonded in Y are selected, identically or differently on each occurrence, from the group consisting of alkyl groups having 1 to 10 C atoms or aromatic or heteroaromatic ring systems having 5 to 20 aromatic ring atoms, which may in each case be substituted by one or more radicals R¹
R¹ is selected on each occurrence, identically or differently, from the group consisting of H, D, F, Cl, Br, I, CN, NO₂, N(Ar)₂, N(R²)₂, C(=O)Ar, C(=O)R², P(=O)(Ar)₂, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may in each case be substituted by one or more radicals R², where one or more non-adjacent CH₂ groups may be replaced by R²C=CR², C=C, Si(R²)₂, Ge(R²)₂, Sn(R²)₂, C=O, C=S, C=Se, C=NR², P(=O)(R²), SO, SO₂, NR², O, S or CONR² and where one or more H atoms may be replaced by D, F, Cl, Br, I, CN or NO₂, an aromatic or heteroaromatic ring system having 5 to 60 aromatic ring atoms, which may in each case be substituted by one or more radicals R², an aryloxy or heteroaryloxy group having 5 to 60 aromatic ring atoms, which may be substituted by one or more radicals R², or a combination of these systems, where two or more adjacent substituents R may optionally form a monocyclic or polycyclic, aliphatic, aromatic or heteroaromatic ring system, which may be substituted by one or more radicals R²;
R² is selected from the group consisting of H, D, F, CN, an aliphatic hydrocarbon radical having 1 to 20 C atoms, an aromatic or heteroaromatic ring system having 5 to 30 aromatic ring atoms, in which one or more H atoms may be replaced by D, F, Cl, Br, I or CN, where two or more adjacent substituents R² may form a mono- or polycyclic, aliphatic, aromatic or hetero-aromatic ring system with one another;
Ar is on each occurrence, identically or differently, an aromatic or heteroaromatic ring system having 5-30 aromatic ring atoms, which may be substituted by one or more non-aromatic radicals R²; two radicals Ar which are bonded to the same N atom or P atom may also be bridged to one another here by a single bond or a bridge selected from N(R²), C(R²)₂ or O;
L is a divalent or polyvalent straight-chain alkylene, alkylidene, alkyleneoxy or thioalkyleneoxy group having 1 to 40 C atoms or a branched or cyclic alkylene, alkylidene, alkyleneoxy or thioalkyleneoxy group having 3 to 40 C atoms or an alkenylene or alkynylene group having 2 to 40 C atoms, which may be substituted by in each case one or more radicals R¹, where one or more non-adjacent CH₂ groups may be replaced by -R¹C=CR¹-, -C=C-, Si(R¹)₂, Ge(R¹)₂, Sn(R¹)₂, C=O, C=S, C=Se, C=NR¹, P(=O)R¹, S=O, SO₂, -O-, -S- or -CONR¹- and where one or more H atoms may be replaced by D, F, Cl, Br, I, CN or NO₂, or an at least divalent aromatic or heteroaromatic ring system having 5 to 80 aromatic ring atoms, which may be substituted by one or more radicals R¹, or P(R¹)₃₋ₚ, P(=O)(R¹)_{3-P}, C(R¹)_{4-P}, Si(R¹)₄₋ₚ, N(Ar)₃₋ₚ or a combination of two, three, four or five of these systems; or L is a chemical bond;
n is on each occurrence, identically or differently, 0 or 1, where, for n = 0, a hydrogen or radical R¹ is present instead of Y, with the proviso that at least one index n = 1 per unit;
p is 2, 3, 4, 5 or 6, with the proviso that p is not greater than the maximum valence of L;
**characterised in that** at least one radical R stands for one of the groups of the following formula (3), 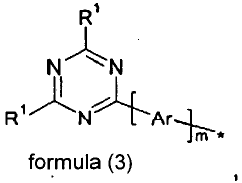 and/or **in that** at least one group L stands for a group of the following formulae (10) or (11): 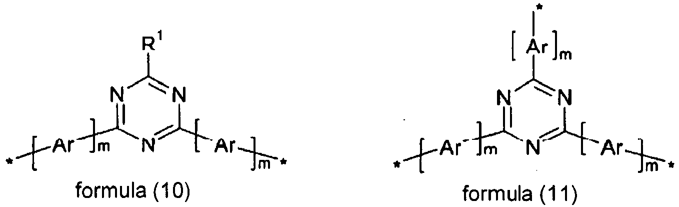 where R¹ stands for an aromatic or heteroaromatic ring system having 5 to 20 aromatic ring atoms which contains no condensed aromatic ring systems having more than 10 aromatic ring atoms and which may in each case be substituted by one or more radicals R², and the other symbols used have the meanings given above, and the index m stands for 0 or 1; * here indicates the position of the bonding of the group of the formule (3), (10) or (11).

2. Organic electroluminescent device according to Claim 1, where the compound of the formula (17) or (18) is selected from the compounds of the formulae (20), (21), (23) or (24), 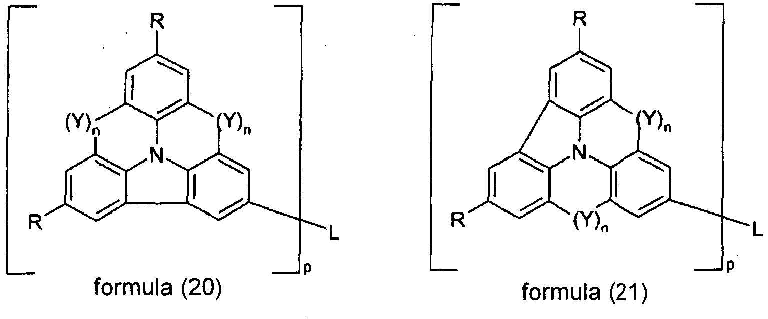 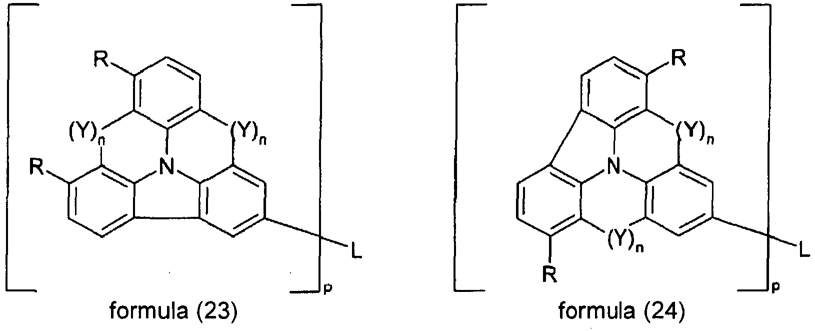 where Y stands, identically or differently on each occurrence, for C(R)₂, the C atoms drawn as unsubstituted may also be substituted by D instead of H, and the other symbols and indices have the meanings given in Claim 1.

3. Organic electroluminescent device according to Claim 1 or 2, **characterised in that** L is an at least divalent straight-chain alkylene group having 1 to 10 C atoms or a branched or cyclic alkylene group having 3 to 10 C atoms, which may be substituted by in each case one or more radicals R¹, where one or more H atoms may be replaced by D or F, or an at least divalent aromatic or heteroaromatic ring system having 5 to 24 aromatic ring atoms, which may be substituted by one or more radicals R¹; or L is a chemical bond; or L is a group of one of the formulae (10) or (11).

4. Organic electroluminescent device according to one or more of Claims 1 to 3, **characterised in that** R is selected, identically or differently on each occurrence, from the group consisting of H, D, F, Cl, Br, CN, N(Ar)₂, C(=O)Ar, a straight-chain alkyl or alkoxy group having 1 to 10 C atoms or a branched or cyclic alkyl or alkoxy group having 3 to 10 C atoms or an alkenyl or alkynyl group having 2 to 10 C atoms, which may in each case be substituted by one or more radicals R¹, where one or more non-adjacent CH₂ groups may be replaced by O and where one or more H atoms may be replaced by D or F, an aromatic or heteroaromatic ring system having 5 to 30 aromatic ring atoms, which may in each case be substituted by one or more radicals R¹, an aryloxy or heteroaryloxy group having 5 to 30 aromatic ring atoms, which may be substituted by one or more radicals R¹, or a combination of these systems, where at least one radical R represents a group of the formula (3); with the proviso that groups R which are bonded in Y are selected, identically or differently on each occurrence, from the group consisting of alkyl groups having 1 to 10 C atoms or aromatic or heteroaromatic ring systems having 5 to 20 aromatic ring atoms, which may in each case be substituted by one or more radicals R¹.

5. Organic electroluminescent device according to one or more of Claims 1 to 4, where the compound of the formula (17) or (18) is selected from the compounds of the formulae (41), (42), (44), (45), (47) to (50), (52), (53), (55), (57) and (58), 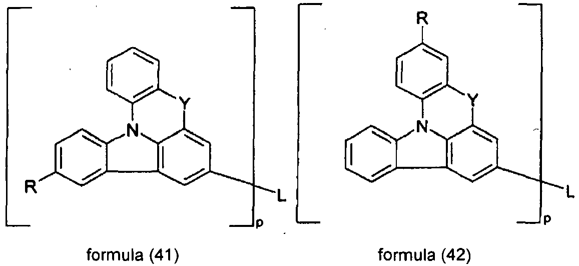 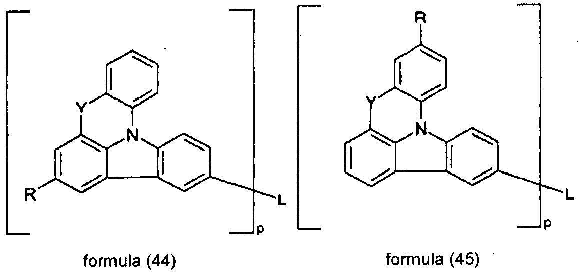 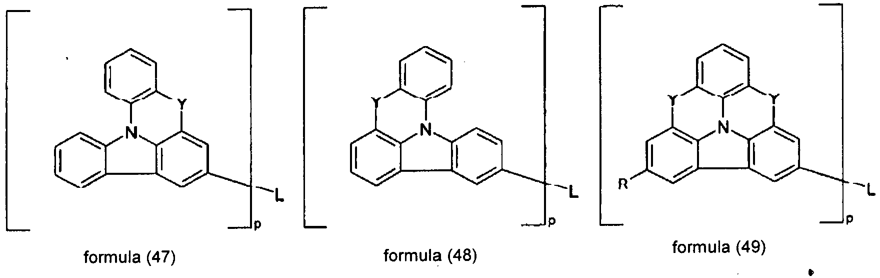 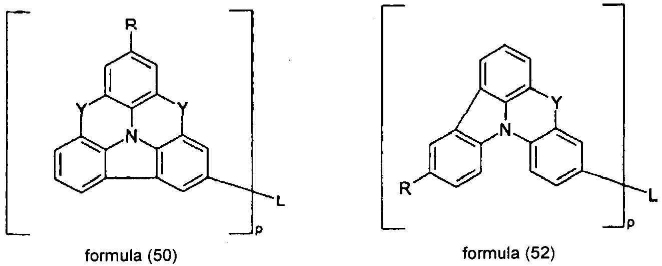 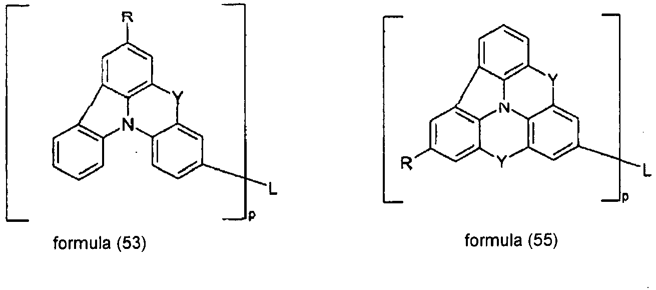 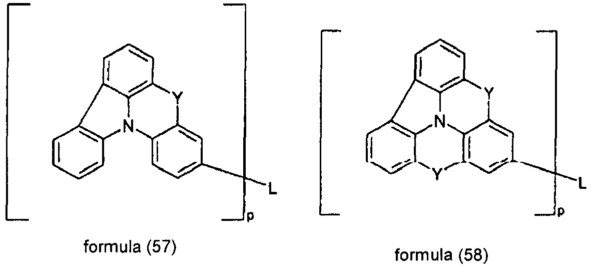 where R represents a group of the formula (3) and where L in formula (47), (48), (57) or (58) represents a group of one of the formulae (10) or (11), Y stands, identically or differently on each occurrence, for C(R)₂, where R which is bonded in the C(R)₂ group stands, identically or differently on each occurrence, for an alkyl group having 1 to 10 C atoms or an aromatic or heteroaromatic ring system having 5 to 18 aromatic ring atoms and preferably at least one of the radicals R in these groups represents an aromatic or heteroaromatic ring system having 5 to 18 aromatic ring atoms, which may in each case be substituted by one or more radicals R¹; furthermore, the C atoms drawn as unsubstituted may also be substituted by D instead of H, and the other symbols and indices have the meanings given in Claim 1.

6. Organic electroluminescent device according to one or more of Claims 1 to 4, **characterised in that** the structures of the formulae (17), (18), (20) and (21) each contain a radical R in the position para to the central atom X, where the substituents R in the para position of X which do not stand for a group of the formula (3) stand for an alkyl group having 1 to 10 C atoms, in particular having 1 to 4 C atoms, or for an aromatic or heteroaromatic ring system having 5 to 24 aromatic ring atoms, which may be substituted by one or more radicals R¹, in particular for a phenyl group, which may be substituted by one or more radicals R¹.

7. Organic electroluminescent device according to one or more of Claims 1 to 6, **characterised in that**, in the structures of the formulae (3), (10) and (11), the symbol R¹ stands for phenyl, ortho-, meta- or para-biphenyl, ortho-, meta- or para-terphenyl, quaterphenyl or 1- or 2-naphthyl, which may in each case be substituted by one or more radicals R².

8. Organic electroluminescent device according to one or more of Claims 1 to 7, **characterised in that** the group Ar in the formulae (3), (10) and (11) stands for an aromatic or heteroaromatic ring system having 5 to 20 aromatic ring atoms which contains no condensed aromatic ring systems having more than 10 aromatic ring atoms and which may be substituted by one or more non-aromatic radicals R¹.

9. Use of a compound of the formula (17) or formula (18), as defined in one or more of Claims 1 to 8, as matrix material for phosphorescent emitters in an organic electroluminescent device.

## Revendications

1. Dispositif électroluminescent organique qui contient un composé de la formule (17) ou de la formule (18) en tant que matériau de matrice pour des émetteurs phosphorescents : 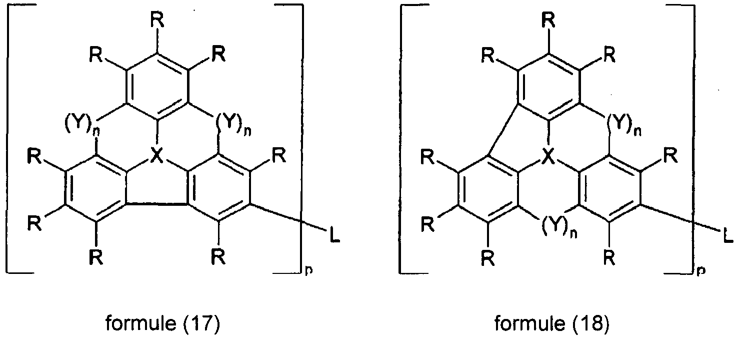 dans lesquelles ce qui suit s'applique aux symboles et indices utilisés :
X est, pour chaque occurrence, N ;
Y est, de manière identique ou différente pour chaque occurrence, C(R)₂ ;
R est sélectionné pour chaque occurrence, de manière identique ou différente, parmi le groupe qui est constitué par H, D, F, Cl, Br, I, CN, NO₂, N(Ar)₂, N(R¹)₂, C(=O)Ar, C(=O)R¹, P(=O)(Ar)₂, un groupe alkyle, alcoxy ou thioalkyle en chaîne droite qui comporte 1 à 40 atome(s) de C ou un groupe alkyle, alcoxy ou thioalkyle ramifié ou cyclique qui comporte 3 à 40 atomes de C ou un groupe alkényle ou alkynyle qui comporte 2 à 40 atomes de C, lequel peut, dans chaque cas, être substitué par un radical ou plusieurs radicaux R¹, où un ou plusieurs groupe(s) CH₂ non adjacents peut/peuvent être remplacé(s) par R¹C=CR¹, C=C, Si(R¹)₂, Ge(R¹)₂, Sn(R¹)₂, C=O, C=S, C=Se, C=NR¹, P(=O)(R¹), SO, SO₂, NR¹, O, S ou CONR¹ et où un ou plusieurs atome(s) de H peut/peuvent être remplacé(s) par D, F, Cl, Br, I, CN ou NO₂, un système de cycle aromatique ou hétéroaromatique qui comporte 5 à 80 atomes de cycle aromatique, lequel peut, dans chaque cas, être substitué par un radical ou plusieurs radicaux R¹, un groupe aryloxy ou hétéroaryloxy qui comporte 5 à 60 atomes de cycle aromatique, lequel peut être substitué par un radical ou plusieurs radicaux R¹, ou une combinaison de ces systèmes, où deux substituants R adjacents ou plus peuvent en option former un système de cycle aliphatique, aromatique ou hétéroaromatique monocyclique ou polycyclique, lequel peut être substitué par un radical ou par plusieurs radicaux R¹, étant entendu que des groupes R qui sont liés sur Y sont sélectionnés, de manière identique ou différente pour chaque occurrence, parmi le groupe qui est constitué par des groupes alkyle qui comportent 1 à 10 atome(s) de C ou par des systèmes de cycle aromatique ou hétéroaromatique qui comportent 5 à 20 atomes de cycle aromatique, lesquels peuvent dans chaque cas être substitués par un radical ou plusieurs radicaux R¹,
R¹ est sélectionné pour chaque occurrence, de manière identique ou différente, parmi le groupe qui est constitué par H, D, F, Cl, Br, I, CN, NO₂, N(Ar)₂, N(R²)₂, C(=O)Ar, C(=O)R², P(=O)(Ar)₂, un groupe alkyle, alcoxy ou thioalkyle en chaîne droite qui comporte 1 à 40 atome(s) de C ou un groupe alkyle, alcoxy ou thioalkyle ramifié ou cyclique qui comporte 3 à 40 atomes de C ou un groupe alkényle ou alkynyle qui comporte 2 à 40 atomes de C, lequel peut, dans chaque cas, être substitué par un radical ou plusieurs radicaux R², où un ou plusieurs groupe(s) CH₂ non adjacents peut/peuvent être remplacé(s) par R²C=CR², C=C, Si(R²)₂, Ge(R²)₂, Sn(R²)₂, C=O, C=S, C=Sₑ, C=NR², P(=O)(R²), SO, SO₂, NR², O, S ou CONR² et où un ou plusieurs atome(s) de H peut/peuvent être remplacé(s) par D, F, Cl, Br, I, CN ou NO₂, un système de cycle aromatique ou hétéroaromatique qui comporte 5 à 60 atomes de cycle aromatique, lequel peut, dans chaque cas, être substitué par un radical ou plusieurs radicaux R², un groupe aryloxy ou hétéroaryloxy qui comporte 5 à 60 atomes de cycle aromatique, lequel peut être substitué par un radical ou plusieurs radicaux R², ou une combinaison de ces systèmes, où deux substituants R adjacents ou plus peuvent en option former un système de cycle aliphatique, aromatique ou hétéroaromatique monocyclique ou polycyclique, lequel peut être substitué par un radical ou plusieurs radicaux R² ;
R² est sélectionné parmi le groupe qui est constitué par H, D, F, CN, un radical hydrocarbone aliphatique qui comporte 1 à 20 atome(s) de C, un système de cycle aromatique ou hétéroaromatique qui comporte 5 à 30 atomes de cycle aromatique, où un ou plusieurs atome(s) de H peut/peuvent être remplacé(s) par D, F, Cl, Br, I ou CN, où deux substituants R² adjacents ou plus peuvent former un système de cycle aliphatique, aromatique ou hétéroaromatique monocyclique ou polycyclique l'un avec l'autre ou les uns avec les autres ;
Ar est pour chaque occurrence, de manière identique ou différente, un système de cycle aromatique ou hétéroaromatique qui comporte 5 à 30 atomes de cycle aromatique, lequel peut être substitué par un radical ou plusieurs radicaux non aromatique(s) R² ; deux radicaux Ar qui sont liés au même atome de N ou de P peuvent également être pontés l'un à l'autre ici au moyen d'une liaison simple ou au moyen d'un pont qui est sélectionné parmi N(R²), C(R²)₂ ou O ;
L est un groupe alkylène, alkylidène, alkylèneoxy ou thioalkylèneoxy en chaîne droite divalent ou polyvalent qui comporte 1 à 40 atome(s) de C ou un groupe alkylène, alkylidène, alkylèneoxy ou thioalkylèneoxy ramifié ou cyclique qui comporte 3 à 40 atomes de C ou un groupe alkénylène ou alkynylène qui comporte 2 à 40 atomes de C, lequel peut être substitué par, dans chaque cas, un radical ou plusieurs radicaux R¹, où un ou plusieurs groupe(s) CH₂ non adjacents peut/peuvent être remplacé(s) par -R¹C=CR¹-, -C=C-, Si(R¹)₂, Ge(R¹)₂, Sn(R¹)₂, C=O, C=S, C=Se, C=NR¹, P(=O)R¹, S=O, SO₂, -O-, -S- ou -CONR¹- et où un ou plusieurs atome(s) de H peut/peuvent être remplacé(s) par D, F, CI, Br, I, CN ou NO₂, ou un système de cycle aromatique ou hétéroaromatique au moins divalent qui comporte 5 à 80 atomes de cycle aromatique, lequel peut être substitué par un radical ou plusieurs radicaux R¹, ou P(R¹)₃₋ₚ, P(=O)(R¹)₃₋ₚ, C(R¹)₄₋ₚ, Si(R¹)₄₋ₚ, N(Ar)₃₋ₚ ou une combinaison de deux, trois, quatre ou cinq de ces systèmes ; ou L est une liaison chimique ;
n est pour chaque occurrence, de manière identique ou différente, 0 ou 1, où, pour n = 0, un hydrogène ou un radical R¹ est présent en lieu et place de Y, étant entendu qu'au moins un indice n = 1 par unité ;
p est 2, 3, 4, 5 ou 6, étant entendu que p n'est pas supérieur à la valence maximum de L ;
**caractérisé en ce qu'**au moins un radical R représente l'un des groupes de la formule (3) qui suit : 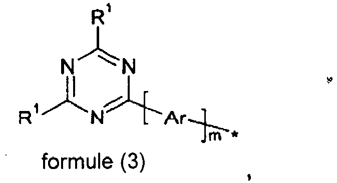 et/ou **en ce qu'**au moins un groupe L représente un groupe des formules (10) ou (11) qui suivent: 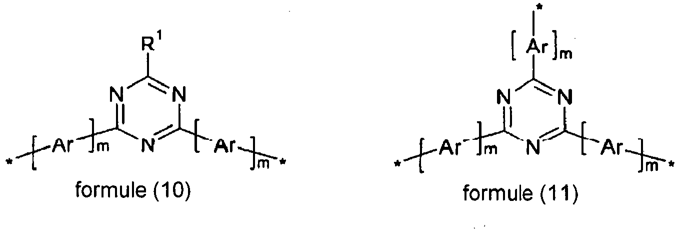 dans lesquelles R¹ représente un système de cycle aromatique ou hétéroaromatique qui comporte 5 à 20 atomes de cycle aromatique, lequel ne contient pas de systèmes de cycle aromatique condensés qui comportent plus de 10 atomes de cycle aromatique et lequel peut, dans chaque cas, être substitué par un radical ou plusieurs radicaux R², et les autres symboles utilisés présentent les significations données ci avant, et l'indice m représente 0 ou 1; * ici indique la position de la liaison du groupe de la formule (3), (10) ou (11).

2. Dispositif électroluminescent organique selon la revendication 1, dans lequel le composé de la formule (17) ou (18) est sélectionné parmi les composés des formules (20), (21), (23) ou (24) : 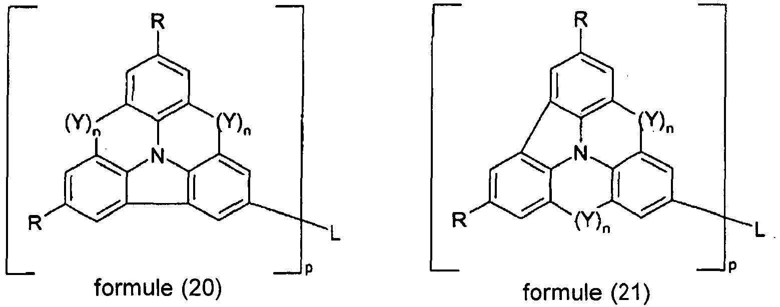 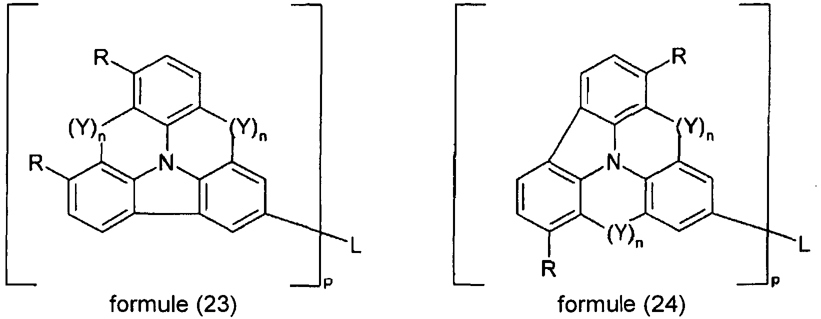 dans lesquelles Y représente, de manière identique ou différente pour chaque occurrence, C(R)₂, les atomes de C qui sont représentés comme étant non substitués peuvent également être substitués par D en lieu et place de H, et les autres symboles et indices présentent les significations qui sont données selon la revendication 1.

3. Dispositif électroluminescent organique selon la revendication 1 ou 2, **caractérisé en ce que** L est un groupe alkylène en chaîne droite au moins divalent qui comporte 1 à 10 atome(s) de C ou un groupe alkylène ramifié ou cyclique qui comporte 3 à 10 atomes de C, lequel peut être substitué par, dans chaque cas, un radical ou plusieurs radicaux R¹, où un ou plusieurs atome(s) de H peut/peuvent être remplacé(s) par D ou F, ou un système de cycle aromatique ou hétéroaromatique au moins divalent qui comporte 5 à 24 atomes de cycle aromatique, lequel peut être substitué par un radical ou plusieurs radicaux R¹ ; ou L est une liaison chimique ; ou L est un groupe d'une des formules (10) ou (11).

4. Dispositif électroluminescent organique selon une ou plusieurs des revendications 1 à 3, **caractérisé en ce que** R est sélectionné, de manière identique ou différente pour chaque occurrence, parmi le groupe qui est constitué par H, D, F, Cl, Br, CN, N(Ar)₂, C(=O)Ar, un groupe alkyle ou alcoxy en chaîne droite qui comporte 1 à 10 atome(s) de C ou un groupe alkyle ou alcoxy ramifié ou cyclique qui comporte 3 à 10 atomes de C ou un groupe alkényle ou alkynyle qui comporte 2 à 10 atomes de C, lequel peut, dans chaque cas, être substitué par un radical ou plusieurs radicaux R¹, où un ou plusieurs groupe(s) CH₂ non adjacents peut/peuvent être remplacé(s) par O et où un ou plusieurs atome(s) de H peut/peuvent être remplacé(s) par D ou F, un système de cycle aromatique ou hétéroaromatique qui comporte 5 à 30 atomes de cycle aromatique, lequel peut, dans chaque cas, être substitué par un radical ou plusieurs radicaux R¹, un groupe aryloxy ou hétéroaryloxy qui comporte 5 à 30 atomes de cycle aromatique, lequel peut être substitué par un radical ou plusieurs radicaux R¹, ou une combinaison de ces systèmes, où au moins un radical R représente un groupe de la formule (3) ; étant entendu que des groupes R qui sont liés sur Y sont sélectionnés, de manière identique ou différente pour chaque occurrence, parmi le groupe qui est constitué par des groupes alkyle qui comportent 1 à 10 atome(s) de C ou par des systèmes de cycle aromatique ou hétéroaromatique qui comportent 5 à 20 atomes de cycle aromatique, lesquels peuvent, dans chaque cas, être substitués par un radical ou plusieurs radicaux R¹.

5. Dispositif électroluminescent organique selon une ou plusieurs des revendications 1 à 4, dans lequel le composé de la formule (17) ou (18) est sélectionné parmi les composés des formules (41), (42), (44), (45), (47) à (50), (52), (53), (55), (57) et (58) : 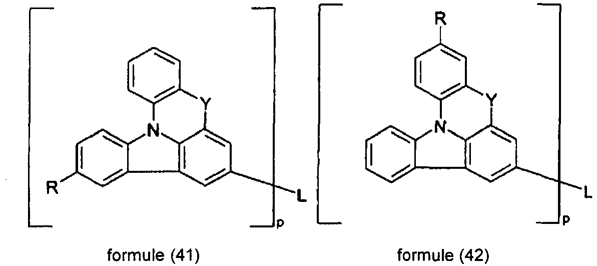 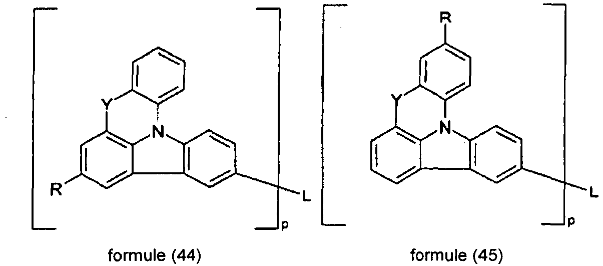 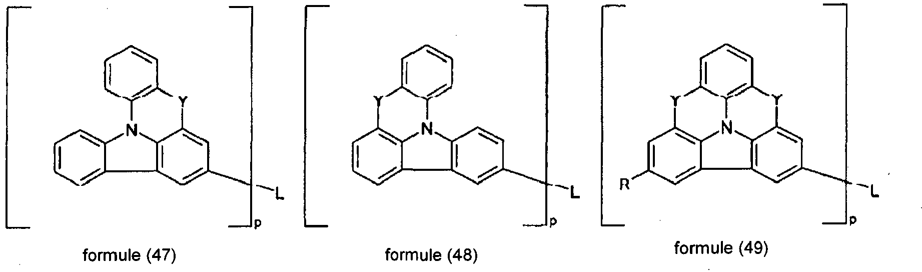 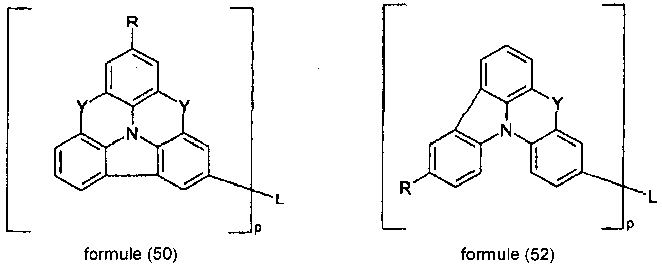 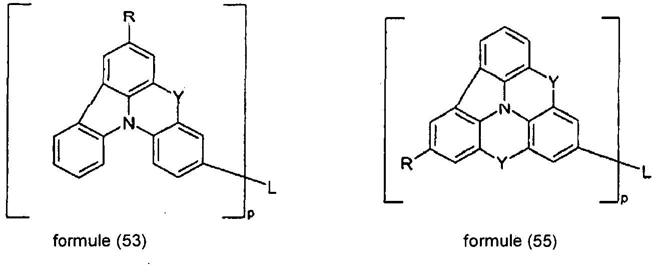 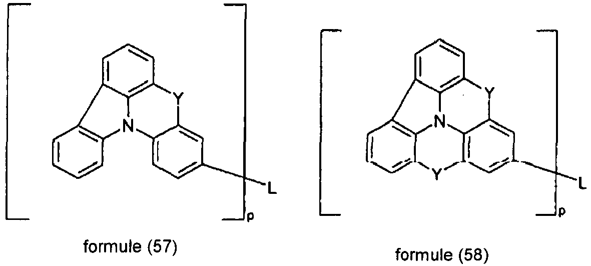 dans lesquelles R représente un groupe de la formule (3) et dans lesquelles L dans les formules (47), (48), (57) ou (58) représente un groupe de l'une des formules (10) ou (11), Y représente, de manière identique ou différente pour chaque occurrence, C(R)₂, dans lesquelles R qui est lié dans le groupe C(R)₂ représente, de manière identique ou différente pour chaque occurrence, un groupe alkyle qui comporte 1 à 10 atome(s) de C ou un système de cycle aromatique ou hétéroaromatique qui comporte 5 à 18 atomes de cycle aromatique et de façon préférable, au moins l'un des radicaux R dans ces groupes représente un système de cycle aromatique ou hétéroaromatique qui comporte 5 à 18 atomes de cycle aromatique, lequel peut, dans chaque cas, être substitué par un radical ou plusieurs radicaux R¹ ; qui plus est, les atomes de C représentés comme étant non substitués peuvent également être substitués par D en lieu et place de H, et les autres symboles et indices présentent les significations qui sont données selon la revendication 1.

6. Dispositif électroluminescent organique selon une ou plusieurs des revendications 1 à 4, **caractérisé en ce que** les structures des formules (17), (18), (20) et (21) contiennent chacune un radical R au niveau de la position para sur l'atome central X, où les substituants R au niveau de la position para de X qui ne représentent pas un groupe de la formule (3) représentent un groupe alkyle qui comporte 1 à 10 atome(s) de C, en particulier qui comporte 1 à 4 atome(s) de C, ou un système de cycle aromatique ou hétéroaromatique qui comporte 5 à 24 atomes de cycle aromatique, lequel peut être substitué par un radical ou plusieurs radicaux R¹, en particulier un groupe phényle, lequel peut être substitué par un radical ou plusieurs radicaux R¹.

7. Dispositif électroluminescent organique selon une ou plusieurs des revendications 1 à 6, **caractérisé en ce que**, dans les structures des formules (3), (10) et (11), le symbole R¹ représente phényle, ortho-, méta- ou para-biphényle, ortho-, méta- ou para-terphényle, quaterphényle ou 1- ou 2-naphtyle, lequel peut, dans chaque cas, être substitué par un radical ou plusieurs radicaux R².

8. Dispositif électroluminescent organique selon une ou plusieurs des revendications 1 à 7, **caractérisé en ce que** le groupe Ar dans les formules (3), (10) et (11) représente un système de cycle aromatique ou hétéroaromatique qui comporte 5 à 20 atomes de cycle aromatique, lequel ne contient pas de systèmes de cycle aromatique condensés qui comportent plus de 10 atomes de cycle aromatique et lequel peut être substitué par un radical ou plusieurs radicaux R¹ non aromatiques.

9. Utilisation d'un composé de la formule (17) ou de la formule (18), comme défini selon une ou plusieurs des revendications 1 à 8, en tant que matériau de matrice pour des émetteurs phosphorescents dans un dispositif électroluminescent organique.
